# EUROPEAN PATENT SPECIFICATION

(11) **EP 3 663 401 B1**
(45) Date of publication and mention of the grant of the patent: **21.09.2022**
(21) Application number: 19160132.7
(22) Date of filing: 28.02.2019
(51) Int. Cl.: C12N 15/10

(54) **PURIFICATION PROCESS FOR BIOLOGICAL MOLECULES SUCH AS PLASMID DNA USING ANIONIC EXCHANGE CHROMATOGRAPHY**
REINIGUNGSVERFAHREN FÜR BIOLOGISCHE MOLEKÜLE WIE PLASMID-DNA UNTER VERWENDUNG VON ANIONENAUSTAUSCHCHROMATOGRAFIE
PROCÉDÉ DE PURIFICATION DE MOLÉCULES BIOLOGIQUES TELLES QU'UN ADN PLASMIDIQUE À L'AIDE D'UNE CHROMATOGRAPHIE À ÉCHANGE D'ANIONS

(43) Date of publication of application: 10.06.2020
(73) Proprietor: Lonza Ltd, 3930 Visp (CH)
(72) Inventor: Zurbriggen, Andreas, 3911 Ried-Brig (CH); Cosandey, Ludovic, 3930 Visp (CH); Balmer, Yves, 1950 Sion (CH)
(74) Representative: Eder, Michael

(56) References cited:
- EP-A1- 2 088 196
- SYREN ET AL: "Milligram scale parallel purification of plasmid DNA using anion-exchange membrane capsules and a multi-channel peristaltic pump", JOURNAL OF CHROMATOGRAPHY B: BIOMEDICAL SCIENCES & APPLICATIONS, ELSEVIER, AMSTERDAM, NL, vol. 856, no. 1-2, 27 August 2007 (2007-08-27), pages 68-74, XP022215588, ISSN: 1570-0232, DOI: 10.1016/J.JCHROMB.2007.05.033
- FRANCO-MEDRANO DIANA IVONNE ET AL: "Plasmid pVAX1-NH36 purification by membrane and bead perfusion chromatography", BIOPROCESS AND BIOSYSTEMS ENGINEERING, SPRINGER, DE, vol. 40, no. 3, 2 December 2016 (2016-12-02), pages 463-471, XP036156696, ISSN: 1615-7591, DOI: 10.1007/S00449-016-1714-6 [retrieved on 2016-12-02]
- ÂNGELA SOUSA ET AL: "Advances in chromatographic supports for pharmaceutical-grade plasmid DNA purification : Liquid Chromatography", JOURNAL OF SEPARATION SCIENCE., vol. 35, no. 22, 7 September 2012 (2012-09-07), pages 3046-3058, XP55595944, DE ISSN: 1615-9306, DOI: 10.1002/jssc.201200307
- CERNIGOJ URH ET AL: "A multimodal histamine ligand for chromatographic purification of plasmid DNA", JOURNAL OF CHROMATOGRAPHY A, ELSEVIER, AMSTERDAM, NL, vol. 1281, 23 January 2013 (2013-01-23), pages 87-93, XP028981292, ISSN: 0021-9673, DOI: 10.1016/J.CHROMA.2013.01.058
- DIANA BICHO ET AL: "Effect of chromatographic conditions and plasmid DNA size on the dynamic binding capacity of a monolithic support : Liquid Chromatography", JOURNAL OF SEPARATION SCIENCE., vol. 37, no. 17, 1 September 2014 (2014-09-01), pages 2284-2292, XP55595099, DE ISSN: 1615-9306, DOI: 10.1002/jssc.201400127
- EON-DUVAL A ET AL: "Purification of pharmaceutical-grade plasmid DNA by anion-exchange chromatography in an RNase-free process", JOURNAL OF CHROMATOGRAPHY B, ELSEVIER, AMSTERDAM, NL, vol. 804, no. 2, 25 May 2004 (2004-05-25), pages 327-335, XP004501590, ISSN: 1570-0232, DOI: 10.1016/J.JCHROMB.2004.01.033

## Description

### FIELD OF THE INVENTION

The present invention provides new, improved methods for the purification or isolation of plasmid DNA (pDNA). The method includes the simple and effective removal of impurities such as RNA, genomic DNA, proteins, cellular fractions, or combinations thereof. The novel methods of the present invention are particularly suitable for large-scale production plants and provide for purified pDNA with excellent quality and good yields, while also allowing for faster processing times and reduced costs.

The purified pDNA obtained by the method of the present invention is particularly suitable for applications in areas such as synthetic transient production, ex *vivo* gene therapy, RNA therapeutics, DNA vaccines, DNA antibodies, non-viral gene therapy, and viral vectors. The purified pDNA obtained via the method of the present invention may also be used, for example, in vaccines, viral therapy, gene and cell therapy, production of molecules such as RNA and polypeptides *in vivo* or *in vitro,* or in chimeric antigen receptor (CAR) T-cell therapy.

The present invention also describes the novel use of a tulip-shaped vessel which may be favorably used during the clarification of a sample comprising the biological molecule of interest. The tulip-shaped vessel or tank may be advantageously used during phase separation for separating solid components from the mixture containing the biological molecule. More specifically, the present invention provides for the novel use of a tulip-shaped vessel or tank for harvesting the lower (i.e. denser) phase of the mixture containing the biological molecule during phase separation.

### BACKGROUND OF THE INVENTION

Plasmid DNA is a small molecule physically separated from chromosomal DNA and can replicate independently. Plasmid DNA typically exists in three different conformations: super-coiled pDNA, open-circular pDNA, and linear pDNA. Given the various therapeutic applications of pDNA, including but not limited to, synthetic transient protein production, ex *vivo* gene therapy, RNA therapeutics, DNA vaccines, DNA antibodies, non-viral gene therapy, and viral vectors, it will be appreciated by those of skill in the art that improved methods of purification or isolation of pDNA are highly desirable.

Having regard to the high negative charge of pDNA, anion exchange chromatography is frequently used in methods for purifying or isolating pDNA. The anion exchange material contains positively charged groups. Thus, anion exchange chromatography separates molecules such as proteins contained in solution according to their charges through the use of an anion exchange material. As those of skill in the art will appreciate, the concentration of the salts used during anion exchange chromatography will vary depending upon the substance that is to be eluted. Examples of typical anion exchange materials include various types of anion exchange membranes, beads, and resins. The proteins bound to the anion exchange material are then eluted through the use of a gradient of increasing concentrations of salt included with an appropriate buffer solution. Thus, in known anion exchange chromatography methods, the loading of the sample is typically carried out with a buffer having a low salt concentration, and the salt concentration is subsequently increased during the elution step.

When purifying pDNA using anion exchange chromatography, particularly on an industrial scale, there are several upstream steps that typically occur prior to loading the solution containing pDNA on the anion exchange column. These steps normally include fed-batch fermentation, cell harvesting and washing, continuous cell lysis, neutralization, RNA removal, flocculate removal depth filtration, plasmid concentration and diafiltration. Subsequently, the pDNA purification process typically includes the use of anion exchange chromatography (AEX), which includes binding the pDNA on the anion exchange material using a salt having low conductivity, followed by elution of the pDNA with a salt having higher conductivity, tangential flow filtration with buffer exchange, and then subjecting the pDNA containing fractions to a further purification step to further enrich super-coiled pDNA, e.g. by PlasmidSelect Xtra^{®} (PSX) chromatography (available, e.g. from GE Healthcare Technology), often followed by a further tangential flow filtration step with a buffer exchange using an end fill buffer. In these known methods for purifying or isolating pDNA using anion exchange chromatography, the elution step is typically achieved by gradually increasing the salt concentration in the elution buffer.

For example, Franco-Medrano et al., Bioprocess and Biosystems Engineering (2017), Vol. 40, pages 463-471 describes a purification of a specific pDNA vector using, *inter alia,* anion exchange chromatography using conventional sodium chloride gradient elution. Similarly, Syren et al., Journal of Chromatography B (2007), Vol. 856, pages 68-74 discloses the purification of plasmid DNA using "Sartobind Q" (a commercial anion exchange chromatography material), eluting the plasmid DNA with high concentrations of sodium chloride. Also, Eon-Duval et al., Journal of Chromatography B, 2004, vol. 804(2), pages 327-335 describe a method for the purification of pharmaceutical-grade plasmid DNA from cell culture employing an anion-exchange chromatography step using conventional salt gradient elution with sodium chloride.

Following the initial binding and elution steps, further steps are required in order to obtain the pDNA in acceptable purity and yield, as mentioned above. Even further, existing methods of purifying pDNA are known to result in poor selectivity and poor recovery. The known methods are thus unable to provide efficient and cost-effective separation of the pDNA. It is also worth noting that many of these known methods suffer from the disadvantage of using PEG or other additives, which may not be desired in the preparation of plasmid DNA, as they require additional separation, disposal and quality control steps. These can be difficult, more time consuming and more expensive. As these additional steps add costs to the purification process and increase the time necessary for purification, there exists a need for a method for purification or isolation of pDNA that eliminates the need for many of the additional steps during purification, thereby reducing the amount of time required for purification, without compromising the purity or yield of the pDNA.

As will be appreciated by those of skill in the art, yet another drawback to known processes for the purification of biomolecules (such as pDNA) is the difficulty to apply such methods in large-scale production, particularly large-scale production of pharmaceutical-grade pDNA. There are many existing constraints on large-scale production, for example personal safety issues and hazardous waste considerations. Further, the application of bench-scale purification processes of pDNA on a larger scale almost always results in decreased yield of the purified pDNA. Given these limitations, it would be desirable to provide for a method of purification or isolation of pDNA that not only provides pDNA with improved purity and in high yield, but that may also be used for large-scale production.

### SUMMARY OF THE INVENTION

A first aspect of the present invention relates to a method for isolating or purifying plasmid DNA ("pDNA") of interest from a mixture, the method comprising contacting the mixture comprising the plasmid DNA of interest with an anion exchange (AEX) material in the presence of a solution comprising a kosmotropic salt at a concentration that allows selective binding of the plasmid DNA of interest to the anion exchange material; and eluting the plasmid DNA of interest with an eluent comprising a chaotropic salt at a concentration that provides an eluant containing the purified pDNA. The kosmotropic salt of this method is selected from the group consisting of: ammonium sulfate, ammonium citrate, ammonium phosphate, potassium phosphate, sodium citrate, sodium phosphate, and mixtures thereof, and
wherein the concentration of the kosmotropic salt is greater than about 0.5 M. In some embodiments, the fraction or fractions comprising said plasmid DNA of interest in the eluant is/are optionally collected. The purified plasmid DNA of interest may in some instances be subsequently isolated from any one or all of the collected fractions.

In some embodiments, the salt used at a concentration that allows selective binding of the biomolecule of interest to the AEX material is a kosmotropic salt, for example ammonium citrate, ammonium sulfate, ammonium phosphate, potassium phosphate, sodium citrate, sodium phosphate, or mixtures of said salts.

In some embodiments, the chaotropic salt that is present in the eluent at a concentration that provides an **eluant** containing the purified plasmid DNAis selected from ammonium chloride, potassium chloride, sodium chloride, magnesium sulfate, magnesium chloride, magnesium nitrate, guanidinium hydrochloride, or mixtures thereof.

The plasmid DNA to be purified by the methods of the invention is typically a highly polar / charged biomolecule. It was found that the novel method described herein is particularly suitable for a fast and highly effective purification of plasmid DNA, in particular pharmaceutical-grade super-coiled plasmid DNA (sc pDNA), which must be free from bacterial genomic DNA, RNA, protein and endotoxins.

The present invention also describes an advantageous sample preparation step that can be advantageously used before contacting the mixture containing the plasmid DNA of interest with the AEX material.

In this sample preparation step, solid components (such as cell debris or other materials insoluble in the buffer system) of the mixture comprising the plasmid DNA of interest are conveniently separated by phase separation and/or filtration. In some cases, the removal of the solid components is achieved via a simple two-phase separation, wherein a buffer is added to increase the density of the mixture to be greater than about 1.1 kg/L. The increase in the density of the mixture causes the solid components to float on top, and the liquid part of the mixture (comprising the plasmid DNA of interest) can then be conveniently drained off, without requiring a filtration step (or at least offering a higher filtration capacity due to the significantly reduced solid material content, thereby allowing scalability of the process, i.e., making it useful for large-scale production). This sample preparation step can in some cases be advantageously combined with the novel purification method described herein.

Related to the above, the present invention also describes the use of a tulip-shaped vessel or tank for the above phase separation step. The tulip-shaped vessel was found to be particularly advantageous for the convenient and effective separation of the solid components from the liquid part of the mixture comprising the plasmid DNA of interest.

### BRIEF DESCRIPTION OF THE DRAWINGS

**Figure 1** depicts a schematic view of plasmid vector pUC19 used as a sample biomolecule to be purified in the working examples.
**Figure 2A** - **D** shows agarose gel electrophoresis (AGE) data on the results of a *binding buffer* screening using Sartobind Q as AEX material.
**Figure 3A - D** shows agarose gel electrophoresis (AGE) data on the results of an *elution buffer* screening using Sartobind Q as AEX material.
**Figure 4** depicts a chromatography profile of the AEX chromatography unit (Sartobind Q) zoomed in on the elution gradient.
**Figure 5** shows agarose gel electrophoresis data of fractions from the AEX chromatography step (Sartobind Q).
**Figure 6** shows AEX-HPLC traces from the Sartobind Q elution pool 1 as depicted in Figure 3 (dashed line) and the standard (solid line).
**Figure 7** shows SDS-PAGE analysis data of the load and elution pool from the Sartobind Q chromatography step.
**Figure 8** includes a schematic representation of the primary recovery process including the AEX chromatography step.
**Figure 9** depicts a chromatography profile of the AEX unit using Sartobind Q.

### DETAILED DESCRIPTION OF THE INVENTION

As will be appreciated by those of skill in the art, in view of the various therapeutic applications for plasmid DNA, developing a simple, and scalable method for purifying plasmid DNA with excellent purity and in high quantity is highly desirable, particularly since many known isolation/purification steps that are typically more suited for small-scale production, such as laboratory and bench-scale production, cannot adequately be translated on a larger scale.

The present invention provides methods for isolation or purification of plasmid DNA as defined in the appended claims. In some embodiments, the present invention includes methods of purification or isolation of super-coiled plasmid DNA free from impurities, including open-circular pDNA, linear pDNA, endotoxins, RNA, lipopolysaccharides, genomic DNA, proteins, and/or combinations thereof via use of anion exchange chromatography under defined, unusual conditions. The methods of the present invention are particularly suitable for large-scale isolation or purification of plasmid DNA (in particular super-coiled plasmid DNA), in a limited number of steps, with high purity and excellent yields. In particular, the purification methods of the present invention can advantageously be used for large-scale production for volumes up to 10,000 liters fermentation working volume.

It will be appreciated that the terms "plasmid DNA of interest", "plasmid DNA", "target molecule" are all used synonymously, referring to the molecule that is to be purified from other components or impurities present in the mixture subjected to the purification method.

Impurities include, but are not limited to, host cell proteins, endotoxin, host cell DNA and/or RNA. It will be appreciated that what is considered an impurity can depend on the context in which the methods of the invention are practiced. An "impurity" may or may not be host cell derived, i.e., it may or may not be a host cell impurity.

"Isolating" or "purifying" plasmid DNA means enrichment of the target molecule from other components which the target molecule is initially associated with. Extents of desired and/or obtainable purification are provided herein. Preferably, the methods of the invention result in an about five-fold enrichment, preferably an about 10-fold enrichment, preferably an about 20 fold enrichment, preferably an about 50 fold enrichment, preferably an about 100 fold enrichment, preferably an about 200 fold enrichment, preferably an about 500 fold enrichment, preferably an about 1000 fold enrichment. Alternatively, the degree of purification may be expressed as a percentage of the first component with respect to another component, or with respect to the resultant preparation. Examples of such percentages are provided herein.

The inventors surprisingly found that it is possible to purify plasmid DNA, such as (super-coiled) plasmid DNA, in a quick, simple and straightforward manner. This is achieved by contacting the mixture comprising the plasmid DNA of interest with an anion exchange (AEX) chromatography material under conditions that yield highly selective binding of the biological molecule to be purified, while the other components in the mixture do not bind to the AEX material.

In this regard, the inventors found that loading the mixture comprising the biomolecule to be purified with the AEX material in a solution having a rather high concentration (> 0.5M) of a kosmotropic salt results in a highly specific binding of the desired biomolecule to the AEX material, i.e. most components in the solution are not bound to the AEX material. These loading conditions are believed to be rather unusual for AEX chromatography, wherein the sample to be purified is typically contacted with the AEX material under conditions of low ionic strength, and subsequently eluted by (gradually or stepwise) increasing salt concentration/ionic strength of the elution buffer. In other words, the specific loading conditions identified by the present inventors allow an effective enrichment of the targeting molecule already during the loading phase of the AEX chromatography step, with further purification possible during properly selected elution conditions, as will be described in detail herein below.

Thus, the AEX chromatography step, when employed under the conditions identified by the present inventors, allows separating the desired plasmid DNAfrom essentially all unwanted contaminants in a single purification step. In fact, the conditions found by the inventors even allow to dispense with a number of preparatory, pre-purification steps commonly applied in the purification of the plasmid DNA of interest due to its effective, and highly selective binding of the target molecule to the AEX material, as many of the contaminants simply do not bind under those carefully selected conditions, as will be explained in greater detail herein below.

The present invention generally relates to a method for isolating or purifying a plasmid DNA of interest from a mixture, wherein the method comprises contacting the mixture containing the biological molecule of interest with an anion exchange (AEX) material in the presence of a solution comprising a kosmotropic salt selected from the group consisting of ammonium sulfate, ammonium citrate, ammonium phosphate, potassium phosphate, sodium citrate, sodium phosphate, and mixtures thereof, at a concentration of greater than about 0.5 M, which allows selective binding of the plasmid DNA of interest to the anion exchange material; and eluting the plasmid DNA of interest from the AEX material with an eluent comprising a chaotropic salt at a concentration that provides an eluant containing the purified plasmid DNA of interest.

"Selective binding" in the context of the present invention means that the large majority of the other components in the mixture do not bind to the AEX material when contacted in a solution comprising the kosmotropic salt at the appropriate concentration. One of skill in the art will appreciate that in biology (and chemistry) binding, elution, etc., will never be 100% exclusive, but for the purposes of the present invention, "selective binding" means that at least 70%, 80%, 85%, 90%, or even 95% of the other components ("contaminants") in the presence of the kosmotropic salt solution will not bind to the AEX material upon contact. For the sake of convenience, the contacting of the mixture with the AEX material may also be referred herein as a "loading step" (as commonly used in column chromatography, although the present method is of course not limited to column chromatography), and the kosmotropic salt solution including the mixture to be purified is also referred as "loading buffer" or "loading solution". The kosmotropic salt solution itself may also be referred to herein as "binding buffer".

Typically, the fraction or fractions comprising the purified plasmid DNA of interest in the eluant is/are collected, although this is not always necessary (for example in analytical purification runs where the concentration / purity of the molecule of interest can be measured directly in solution, e.g. by UV spectroscopy). Likewise, in most cases, the plasmid DNA of interest is then isolated from any one or all of the collected fractions for further use, as outlined herein below.

For most biomolecules, isolation does not necessarily mean precipitation and drying, but rather keeping the purified molecule in a, typically buffered, solution that ensures stability against degradation, e.g. in long term storage. The isolation may thus also include concentration, i.e. removal of part of the solvent to increase the concentration of the target molecule. As is well-known in the art, solutions comprising a biomolecule (be it proteins or nucleic acid based biomolecules) can in many cases be advantageously stored in a frozen solution, although in some instances, solvent may also be removed, e.g. by lyophilization.

AEX chromatography is typically characterized by loading the mixture to be purified onto the AEX material in a buffer comprising a salt/buffer with low ionic strength, i.e. at rather low salt concentrations. These conditions allow highly polar and/or charged molecules to bind to the charged groups on the AEX material, while the components having lower polarity / hydrophilicity will not bind under such conditions. The molecule of interest is then typically eluted from the column by successively increasing the concentration of the salt/buffer so that the increased number of ions compete with the binding of the molecules still bound to the AEX material. In order to achieve the desired high degree of purity, a gradient increasing the ionic strength of the elution buffer is typically used for purifications via AEX chromatography. In many cases, however, conventional AEX chromatography does not achieve sufficiently high purity because too many contaminants (especially charged / highly polar contaminants) also bind to the AEX material during the loading phase, and are then eluted at the same time as the molecule of interest.

The inventors have surprisingly found that it is possible to carry out the loading step (i.e. the initial contact of the mixture with the AEX material) in solutions comprising kosmotropic salts at relatively high concentrations (greater than about 0.5 M). *Kosmotropic* salts contribute to the stability and structure of water-water interactions ("order-making") by causing water molecules to favorably interact, which also leads to a stabilization of intramolecular interactions in macromolecules such as proteins. Ionic kosmotropes are characterized by strong solvation energy which typically leads to an increase of the overall cohesiveness of the solution (as often indicated by an increase of the viscosity and density of the solution). In contrast, chaotropic agents/salts (disorder-makers) have the opposite effect, disrupting water structure, increasing the solubility of nonpolar solvent particles, and destabilizing solute aggregates.

The inventors have found that using such a kosmotropic salt in the solution contacted with the AEX material at a sufficiently high concentration leads to a highly selective binding of certain biomolecules (particularly biomolecules having a high charge density such as plasmid DNA), while preventing other components from binding to said AEX material. Thus, in some instances, the "flow-through" of the loading step in a solution comprising a kosmotropic salt at a sufficiently high concentration will already contain most, if not essentially all contaminants from the mixture comprising the biomolecule of interest.

It will be appreciated that the property of being kosmotropic or chaotropic is usually ascribed to the specific ion and not a salt (which comprises a counter ion). Thus, it may well be that a salt may comprise an anion that is known to be kosmotropic, while the counter ion is rather known to be chaotropic. Such salts may (or may not) work in selectively binding the target molecule to the AEX material. However, those of skilled in the art will be able to find out whether a given salt will, at high concentrations, ensure the selective binding of the target molecule on the AEX material by simply carrying out routine experiments.

In most embodiments, elution of the target molecule from the AEX material can then be achieved by an eluant (i.e. a solution or buffer used to detach the components bound to the AEX material) comprising a chaotropic salt in a concentration sufficiently high to elute the biological molecule of interest. The elution phase is thus in many embodiments not materially different from conventional AEX chromatography, but due to the higher specificity in binding to the AEX material during the loading phase, the purity of the eluant is, often significantly, increased.

In the methods of the present invention, the biological molecule to be purified is plasmid DNA. Plasmid DNA often exists in different conformations: apart from the "native" super-coiled (SC) conformation, plasmid DNA may also be present in open circular (OC), or even in linear form. Since super-coiled plasmid DNA (sc pDNA) typically represents the desired (and commercially relevant) conformation for plasmid DNA, the biomolecule of interest in some embodiments of this aspect of the present invention is sc pDNA.

The plasmid DNA to be purified/isolated may typically comprise mammalian DNA, bacterial DNA, non-coding DNA, or viral DNA. In some instances, the plasmid DNA will comprise DNA capable of expressing a polypeptide of interest. The purification method will generally not depend on the size of the plasmid DNA, i.e. minivectors with only 350 bp as well as large plasmid vectors comprising up to 20 genes (35 kbp), and anything in-between may be effectively purified by the method described herein.

The method of the present invention allows the effective purification of plasmid DNA from other contaminants. Accordingly, in certain embodiments, the resulting **purity** of the plasmid DNA is at least 90%, or at least 93%, or at least 95%, or at least 97%, or at least 98% or at least 99%.

In some embodiments, the method even accomplishes the fractionation of super-coiled plasmid DNA from other plasmid DNA forms, such as linear plasmid DNA, open-circular plasmid DNA, and, optionally, other nucleic acid molecules.

Since the plasmid DNA of interest is typically obtained by biological processes (such as cell culture/fermentation processes), the mixture comprising the target molecule will in many embodiments include plasmid DNA (including super-coiled pDNA, open-circular pDNA, and linear pDNA), genomic DNA, RNA, lipopolysaccharides, endotoxins, proteins, or any combination of the foregoing components.

It will be appreciated that the concentration of the kosmotropic salt allowing selective binding of the biological molecule of interest to the AEX material during the loading step typically depends on the nature of the target molecule and the chosen AEX material. In some embodiments, highly selective binding of the target molecule has been achieved with salt concentrations of greater than about 0.5 M, or greater than 0.6 M, or greater than 0.8 M. In certain embodiments, the salt concentration of the solution allowing the selective binding of the target molecule is between about 0.5 M and about 4.0 M, or between about 0.8 M and about 2.0 M, or between about 1.0 M and about 2.0 M, such as 1.5 M.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%.

As noted earlier, kosmotropic salts at the above concentrations are employed for the loading step of the present purification method. In any event, the ideal concentration of said salt for the loading step is dependent on the actual properties of the target molecule and the AEX material, but can be easily determined by one of skill in the art through routine experiments. Once a suitable concentration has been determined, it may be useful for consistent and reproducible binding to equilibrate the AEX material with a buffer comprising the kosmotropic salt at the same concentration as in the loading solution comprising the target molecule to be purified.

In any event, examples of suitable kosmotropic salts include ammonium sulfate, ammonium citrate, ammonium phosphate, potassium phosphate, potassium citrate, sodium citrate, sodium phosphate, or mixtures thereof. For example, it was found that ammonium sulfate is a particularly useful kosmotropic salt for the loading phase when the target molecule to be purified is plasmid DNA.

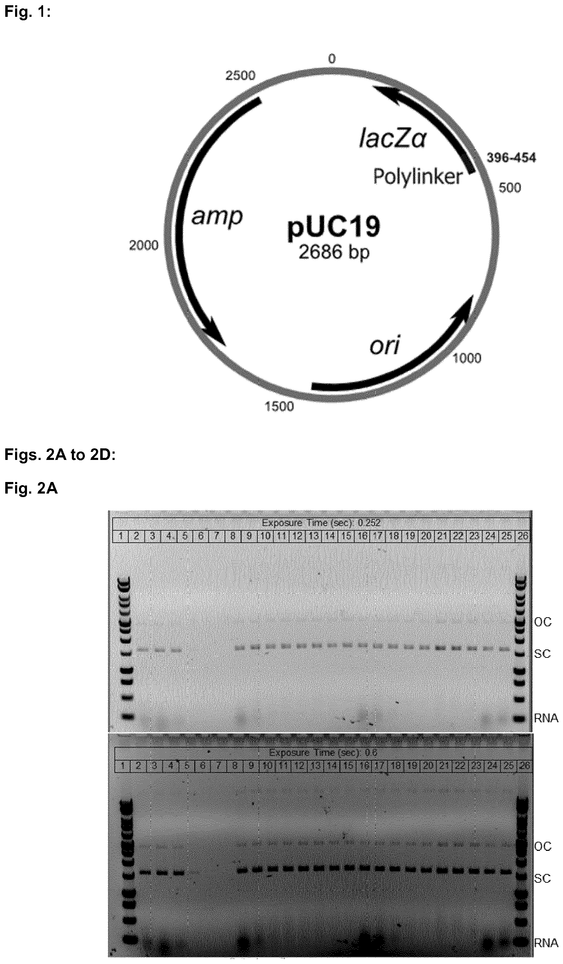

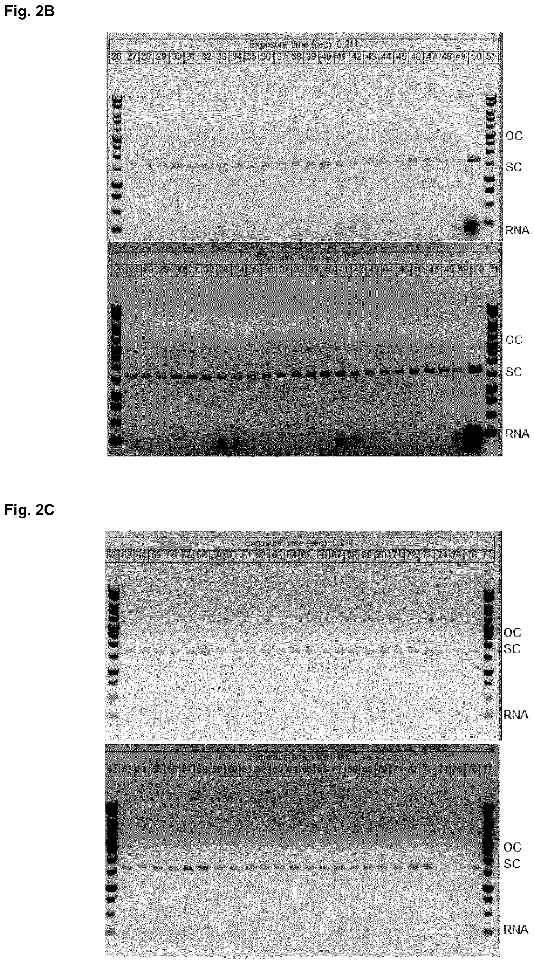

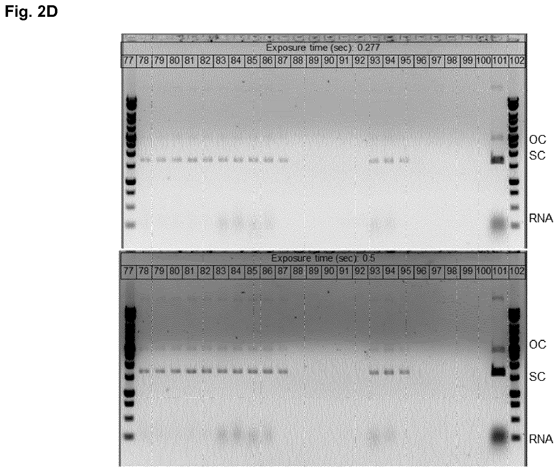

As can be seen from Example 1, loading the mixture onto an optionally pre-equilibrated AEX membrane in a solution comprising high concentrations (> 0.5 M) of ammonium sulfate ensured highly selective binding of the pDNA to the AEX material, while RNA and other contaminants did not bind to the AEX membrane (cf. Fig. 2A and 2B). Accordingly, a useful concentration of ammonium sulfate in the loading solution is in certain embodiments about 0.5 M to about 2.0 M.

One of skill in the art will appreciate that other factors may exert an influence on the binding behavior of the components in the mixture. One of these factors is the pH of the solution. For biomolecules, the available pH range for purifications is naturally rather limited. In order to avoid pH conditions that may negatively affect the stability of the target molecule (e.g. by promoting hydrolysis of the biomolecule), pH values during the loading and elution of the biomolecule of interest typically range between pH 2 and pH 11, although pH values closer to neutral will generally be preferred, especially for biomolecules that are sensitive to degradation upon acidic or alkaline conditions. For example, in some embodiments, the pH of the solution comprising the kosmotropic salt contacted with the AEX material (i.e. the "loading buffer") is between pH 4 and pH 9, or between pH 5 and pH 8, or between pH 6 and pH 10, or between pH 6.0 to 8.0, e.g. around pH 7.0. Those of skill in the art will appreciate that optimal pH conditions may be determined by one of skill in the art by routine experiments.

It will be further appreciated that a pH change, for example during the elution step, may change the binding behavior/strength of the biomolecules bound to the AEX material, and may therefore be used in some embodiments for the controlled and selective release of the target molecule from the AEX material. However, in other instances, the binding of the target molecule to the AEX material will be essentially independent of the pH. For example, the binding of plasmid DNA did not change within a wide pH range (as low as pH 2).

With regard to the conditions selected for elution of the target molecule, the concentration of the chaotropic salt that provides an eluant containing the purified biological molecule of interest is typically about 0.25 M to about 4.0 M, or about 0.4 M to about 3.0 M, or about 0.5 M to 2.0 M. As is well-known in the art, the elution step may include a gradient elution by varying the concentration of the (preferably chaotropic) salt in the eluent, which typically involves increasing (continuously or step-wise) the concentration of the chaotropic salt in the eluent.

In some embodiments, the chaotropic salt for the elution step is selected from the group consisting of: ammonium chloride, potassium chloride, sodium chloride, magnesium sulfate, magnesium chloride, magnesium nitrate, guanidinium hydrochloride, and mixtures thereof. A particularly useful salt, especially for the purification of plasmid DNA, is magnesium sulfate. In these embodiments, the concentration of chaotropic salt, e.g., magnesium sulfate or sodium chloride is typically at least about 0.5M, and may in some instances range from 0.5 M to about 2.0 M or from about 0.5 M to about 1.0 M.

Again, as in the case of the kosmotropic salt contained in the loading buffer, the chaotropic salt and its optimal concentration for the elution of the target molecule depends on the nature of the biological molecule of interest and the specific AEX material chosen for the purification, and can be easily determined by one of skill in the art through routine experimentation (see Examples). Since the AEX material can typically be reused for another purification run, it may be useful to add a high salt concentration elution at the end (e.g. with about 2.0 to 4.0 M NaCl) which will strip essentially any bound material from the AEX material.

In principle, any available AEX materials may be used in the context of the method of the present invention. For example, the method can be applied with weak and with strong AEX set-ups, at high and low ligand density, with different bead chemistries and/or linkers. Suitable AEX materials are for example available as an anion exchange membrane, an anion exchange resin, a three-dimensional microporous hydrogel structure, a packed bed of superporous beads, macroporous beads, a monolith, agarose beads, cross-linked agarose, silica beads, large pore gels, methacrylate-based beads, polystyrene-based beads, cellulose-based beads, dextran-based beads, bisacrylamide-based beads, polyvinylether-based beads, ceramic-based beads, or polymer-based beads. Examples of commercially available AEX materials include, among others, Sartobind Q^{®}, Mustang Q^{®}, Mustang E^{®}, Poros XQ^{®}, Poros HQ^{®}, Nuvia Q^{®}, Capto Q^{®}, Bakerbond PolyQuat^{®} , DEAE Sepharose^{®}, NH2-750F^{®}, Q Sepharose^{®}, Giga Cap^{®} Q-650M, Fractogel^{®} EMD COO, NatriFlo^{®} HD-Q, and 3M^{™} Emphaze^{™} AEX Hybrid Purifier (all registered trademark names).

Particularly for larger, industrial scale purifications, the method may be carried out using AEX membrane chromatography or resin bed chromatography. In some embodiments, the anion exchange membrane has an average pore size of about 3.0 µm to about 5.0 µm, preferably the average pore size is about 3.0 µm.

Alternatively, the method may be carried out using AEX column chromatography. In these embodiments, the anion exchange resin has preferably an average particle diameter from about 30 µm to about 300 µm.

In any event, it will be appreciated by those of skill in the art that the method described herein does not require the AEX material to be in a specific form, i.e. other AEX materials in whatever form, such as the other alternatives mentioned above, may also be employed as the purification agent in the purification method described herein.

Since the method provides excellent purification abilities, it may typically be carried out in the absence of any organic solvents, detergents, glycols, hexamine cobalt, spermidine, and / or polyvinylpyrrolidone, thereby no longer necessitating removal of such agents before providing the final target molecule in isolated form.

While the AEX purification step represents the main aspect of the present invention, it will be appreciated that a purification method for a biomolecule of interest will typically include further method steps, including steps carried out *prior to* the AEX chromatography step, but also, optionally, additional steps carried out *subsequent to* the AEX chromatography step.

As noted earlier, biological molecules of interest such as plasmid DNA are often obtained from cells grown in a so-called cell culture. The biological molecule of interest may in certain cases be obtained from the cell culture in a rather straightforward way when the molecule is secreted by the cells into the surrounding cell culture medium. However, in most cases the target molecule will need to be released from the cells by destroying the cells via cell lysis, which may be accomplished by physical and/or chemical means, as is well-known in the art. Cell lysis will typically yield the target molecule together with a large number of host cell derived water-soluble and insoluble contaminants, such as cell membranes, cell organelles, genomic DNA, RNA and host cell proteins. Thus, a purification of a target molecule will typically require removal of these contaminants, where in particular the solid (i.e. non-soluble) contaminants are removed prior to the AEX chromatography step, typically by mechanical means.

Accordingly, in some embodiments of the present invention the method further comprises separating solid components from the mixture comprising the plasmid DNA of interest *prior to* contacting the mixture with an AEX material.

The removal of solid components from the mixture to be purified may for example be achieved by filtration or phase separation. In some instances, this method step achieves removal of solid components via a two-phase separation, such as a solid-liquid phase separation.

In order to achieve a better separation, the mixture comprising solid components may in some embodiments be modified, for example by increasing the density of the mixture, for example to a density of greater than about 1.1 kg / L. This increase in the density of the mixture results in the solid components (such as cell debris or other precipitated matter) floating on top of the mixture. The solution comprising the plasmid DNA of interest can then be conveniently drained off from the bottom part of the vessel / tank containing the mixture. The adjustment of the density, such as to at least about 1.1 kg / L, therefore yields a mixture where no filtration is required to obtain a clarified mixture that can subsequently be subjected to AEX chromatography. The density of the solution can generally be increased by a variety of operations. For example, a water-soluble salt, a carbohydrate (e.g. glucose, sucrose, glycerol, etc), urea, or other components that increase the density of an aqueous solution. In particular in the context of the purification method of the present invention, it may however be advantageous to employ a kosmotropic salt that is also suitable for the loading step of the AEX chromatography step, such as any of the kosmotropic salts described herein above.

Alternatively, phase separation techniques to remove insoluble contaminants may also be achieved by modifying the density of the mixture in other ways. For example, by adding alcohols (e.g. ethanol, methanol, isopropanol, etc) to the mixture the density would be decreased, causing the solid components to sediment to the bottom of the vessel or tank (optionally sedimentation could be assisted with centrifugation). Another option would be to add liquid non-aqueous compounds to the mixture so as to establish a liquid/liquid two-phase system, e.g., a PEG/salt two-phase system where the insoluble particles will be in the PEG phase and the plasmid DNA in the salt phase.

Furthermore, it will be understood that a light filtration, for example a depth filtration, may in certain cases still be applied for either the liquid, drained-off part of the mixture and/or for the final remaining volume of the mixture (to maximize yields). The optional additional filtration step ensures removal any remaining solid particles in the liquid part of the mixture. In any event, it will be appreciated that such a filtration will be much quicker and will not clog the filter due to the much lower solid content in the mixture. Depth filtration devices are commercially available, for example under the name Clarisolve^{®} depth filter from Merck-Millipore.

Since this method step can also be used in the preparatory phase of other purification methods, the present disclosure also describes the above two phase separations *per se,* i.e., not or not necessarily followed by the AEX chromatography step of the present invention.

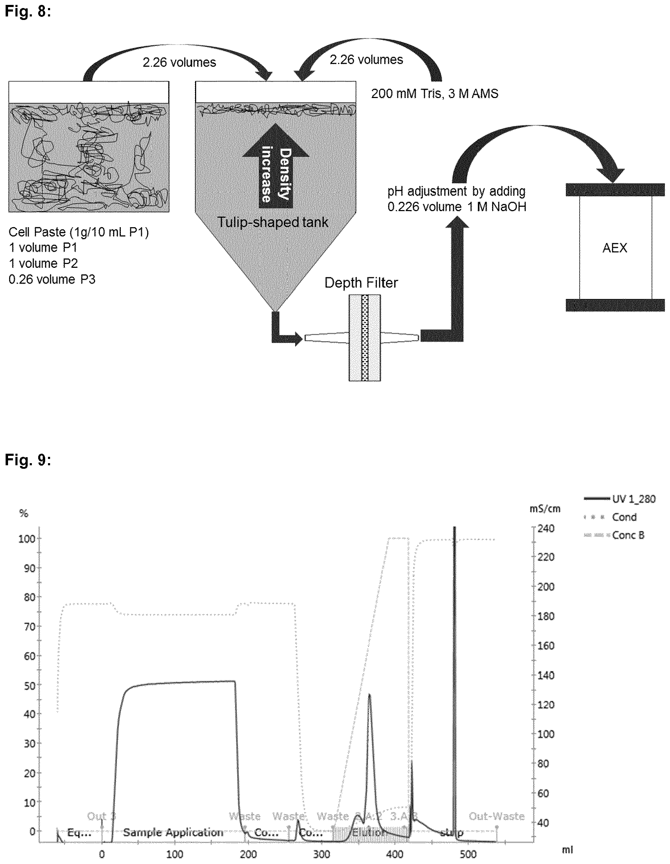

The inventors have also found that a tulip-shaped vessel or tank is particularly useful for the removal of solid components in the mixture, particularly when the density is adjusted to at least about 1.1 kg / L which ensures that the solid components will float on top of the solid/liquid two phase mixture, i.e. where the diameter of the vessel/tank is greater that in the bottom part of the vessel from which the liquid phase is drained (see, for example the tulip-shaped vessel in **Figure 8****).** Accordingly, the use of a tulip-shaped tank or vessel for the above-described two phase separation represents another part of the present disclosure.

The above-described two-phase separation represents a highly effective, fast, reproducible and economic method to prepare the solution comprising the biological molecule of interest for any subsequent purification step, such as the AEX chromatography step of the present invention. Particularly when the density adjustment is already accomplished with a relatively high concentration of the kosmotropic salt as used in the subsequent AEX chromatography step (e.g., ammonium sulfate), the two-phase separation may be the only step (possibly combined with a depth filtration prior to contacting the mixture with the AEX material) required after harvesting the cell supernatant. Since some of the kosmotropic salts act as a buffer, another advantage of adjusting the density of the mixture with said kosmotropic salts is that less neutralization buffer (to prevent possible degradation of the target molecule) is required immediately after cell lysis.

Moreover, the convenient removal of the solid components by simple draining off of the denser solution containing the target molecule is highly advantageous for the subsequent (though optional) depth filtration, not the least since the required filter area is greatly reduced as only a small fraction of the insoluble particles are processed over the filter.

The AEX chromatography method of the present invention may in some embodiments contain further steps after elution of the purified plasmid DNA of interest. For example, in some cases, the method further comprises the step of precipitating the plasmid DNA of interest from the eluant. Precipitation may be achieved by a variety of methods generally known to the art, such as changing the pH of the eluant fractions comprising the target molecule, or adding antisolvents or other additives to the eluant, thereby causing the target molecule to precipitate from the eluant.

After precipitation, the method may in some instances further comprise filtering the eluant by tangential-flow filtration to isolate the precipitated plasmid DNA. For example, in some cases, the average pore size of the filtration membrane used in the tangential-flow filtration step will be ≤ 0.45 µm or even ≤ 0.2 µm.

In some instances, the method may further include the lyophilization of the purified plasmid DNA of interest, optionally in the presence of a carbohydrate that may stabilize the plasmid DNA in lyophilized form during storage. Many mono- or disaccharides can be used for said purpose as is generally known in the art.

In other embodiments, the method will further include dilution, concentration, or buffer exchange of the fraction(s) comprising the purified target molecule. In certain cases, the target molecule may be modified chemically (e.g., biotinylation, methylation, acetylation), or cut into smaller pieces (e.g. digestion by restriction enzymes in the case of nucleic acids).

Depending on the specific purification problem, and source for the target molecule, it may in some cases be useful to further collect the flow-through from the loading step (i.e. the step of contacting the mixture with an AEX material in the presence of the solution comprising the kosmotropic salt at a concentration of greater than about 0.5 M, which allows selective binding of the plasmid DNA to the anion exchange material).

As discussed herein, the AEX chromatography step is capable of selectively binding a plasmid DNA of interest to the AEX material, i.e. most of the unwanted components in the mixture will not bind to the AEX material under these loading conditions. For example, for purification of plasmid DNA from a cell culture, the flow-through may comprise RNA, genomic DNA, proteins, cellular fractions, or combinations thereof. In some embodiments, the flow through will comprise substantially all (i.e. at least 80%, at least 90%, or even at least 95%) RNA that was present in the mixture contacted with the AEX material.

Purification may in some instances be improved by further including a washing step. Thus, in some embodiments the method will further comprise washing the AEX material with a washing buffer solution prior to the elution of the plasmid DNA of interest. In some instances, washing may be continued with additional clean "loading" buffer (i.e. comprising a kosmotropic salt at relatively high concentration). In other instances, a washing buffer solution may comprise a chaotropic salt at a concentration lower than the concentration required for the elution of the biological molecule of interest.

In these cases, the washing buffer may also still comprise the kosmotropic salt (possibly in lower concentration than in the loading buffer), or it may only comprise the chaotropic salt. The chaotropic salt for the washing buffer is preferably selected from the group consisting of: ammonium chloride, potassium chloride, sodium chloride, magnesium sulfate, magnesium chloride, magnesium nitrate, and mixtures thereof. In particular when the chaotropic salt has no buffer capacity, the washing buffer may further comprise a suitable buffer substance to maintain the pH at the desired level. For simplification, the washing buffer will in many cases comprise the same chaotropic salt as in the eluent, although this may not be required in terms of purification efficiency.

It will be further appreciated that, particularly when the purification of the target molecule must yield the product at a very high level of purity, the method of the present invention may further comprise an additional purification step, which typically follows the step of eluting and optionally isolating the biological molecule from the AEX chromatography step. In some embodiments where the target molecule to be purified is super-coiled plasmid DNA, the second purification step may therefore involve the use of a thiophilic aromatic adsorption chromatography medium, available for example from GE Healthcare Life Sciences under the trade name PlasmidSelect Xtra^{®}, or in short "PSX"). This particular resin is known to have excellent selectivity to separate super-coiled plasmid DNA from open circular and/or linear plasmid DNA, and may therefore be used for the final enrichment of the super-coiled conformation of pharmaceutical-grade DNA.

In some embodiments, the method will further comprise, after purification and isolation of the plasmid DNA, a further step of using said plasmid DNA for the expression of a polypeptide of interest encoded by said plasmid DNA. The polypeptide produced via the plasmid DNA will typically be harvested and, optionally, purified. Finally, the method may in some cases even include the formulation of the polypeptide (obtained via expression of the purified plasmid DNA) into a pharmaceutical composition which may optionally comprise one or more pharmaceutically acceptable excipients.

Alternatively, when the plasmid DNA, is intended for pharmaceutical use directly, the method may include the formulation of the nucleic acid into a pharmaceutical composition, which may again optionally comprise one or more pharmaceutically acceptable excipients. Plasmid DNA purified by the method of the present invention may be used as DNA-based vaccine or be used as a "prodrug" wherein the plasmid will then involve the cell's transcription and translation apparatus to biosynthesize the therapeutic entity *in situ.*

In yet other embodiments, the method will further comprise the use of the purified plasmid DNA for the production of RNA, including RNA-based drugs, for the production of shorter oligonucleotides such as siRNA or aptamers (short single-stranded nucleic acid segments (typically 20-60 nucleotides), or for the production of DNAzymes (ribozyme analogs with an RNA backbone replaced by DNA motifs that confer improved biological stability).

A preferred variant of the purification method involving AEX chromatography comprises, prior to said AEX chromatography step, the steps of cell harvesting and washing, cell lysis, neutralization, and flocculate removal prior to contacting the solution comprising the plasmid DNA of interest with the AEX material.

As noted above, the conditions identified for the AEX loading step allows for selective binding of plasmid DNA to the AEX material. Thus, RNA, which is typically also bound to the AEX material under standard, low salt conditions, does not or not substantially bind to the AEX material under the loading conditions described herein, and can thus be quantitatively removed from the target molecule in a single step.

The method described herein thus has the great advantage that RNA does not need to be removed by a calcium chloride (CaCl₂) precipitation step. Avoiding additional purification steps are of course beneficial in terms of yield, costs and duration of the overall purification method. Accordingly, the method is in certain embodiments be further characterized in that it does not comprise a calcium chloride (CaCl₂) precipitation step.

The same is true for proteins which also do not bind to the AEX material under the conditions identified herein. Since open-circular pDNA, linear pDNA and genomic DNA all have different interaction strength with AEX ligands compared to super-coiled plasmid DNA, their levels can be greatly reduced during the AEX chromatography step. Accordingly, the method as described and claimed herein allows for the simple, quick, highly effective and economic purification of plasmid DNA of interest involving essentially only a single chromatographic step involving materials having AEX ligands.

Many additional steps commonly needed in plasmid DNA purification procedures described in the prior art, such as buffer exchange, (multiple) depth filtrations, CaCl₂ precipitation or additional chromatographic steps, such as PSX chromatography, can be avoided (depending on the required content of sc pDNA). Finally, the method described herein allows scaling it for large production plants.

Having described the various aspects of the present invention in general terms, it will be apparent to those of skill in the art that many modifications and slight variations are possible without departing from the scope of the present invention.

### EXAMPLES

### Example 1: Screening of conditions for binding and elution with anion exchange material Sartobind Q for a sample plasmid DNA (pUC19)

All buffers used throughout the process were filtered through 0.2 µm filters before use. E. coli cells harboring the plasmid pUC19 (a schematic description of this plasmid DNA is found in Fig. 1) were grown, lysed and clarified through filtration methods as described in more detail below.

Cells were grown in small scale bioreactors at fed-batch condition to an optical density of 65 - 90, measured at 600 nm (OD₆₀₀). The cells were harvested by centrifugation (4'400 - 4'800, 45 minutes). The cells harvested at the end of cellular growth were resuspended in P1 buffer (50 mM Tris, 10 mM EDTA; pH 7.4). Then P2 buffer (0.2 M NaOH, 1% (w/v) SDS) was added in a 1:1 ratio (v/v), in order to perform alkaline lysis of the cells. The mixture was gently homogenized and left to rest at room temperature for 5 minutes. To stop cell lysis and neutralize the mixture, the appropriate volume of cold P3 buffer (5 M potassium acetate, acetic acid, 4 °C), approximately 25 % in volume of the other buffers, was added. After gentle homogenization, the conditioning buffer containing 3 M Ammonium sulfate was added in a 1:1 ratio (v/v), in order to change the density of the solution, thereby causing the cell debris to float on the upper part of the solution. The solution was then subjected to a final depth filtration (Clarisolve depth filter, Merck-Millipore, with pore sizes from 40 to 0.5 µm) to remove any remaining cell debris, as well as some precipitated genomic DNA and proteins. After filtration, the pH of the filtrated solution was adjusted to pH 7.0 by adding an appropriate volume of 1 M NaOH to the filtrate.

Screening of the binding and elution conditions were performed on a liquid handling robot (Freedom Evo 150; Tecan) and allowed 96 chromatography steps to be run in parallel. Sartobind Q 96 well-plates (Cat#: 991EXQ42GC ----- V, Sartorius) were used for the screening. The load conditioning was performed by changing buffer with the help of Zeba Spin desalting plates (Cat# 89808, ThermoScientific) and the respective binding buffer. The Sartobind Q units were equilibrated with binding buffer before 2.0 mL of the load solution were applied. The membrane was washed with 1 mL of the respective binding buffer and 1 mL of wash buffer (20 mM Na-Phosphate, pH 7.5). Material was eluted by applying 1 mL of elution buffer followed by a strip step with 1 mL of 4 M NaCl. Sample analyses were performed by agarose gel electrophoresis (AGE), where quantities and qualities can be estimated.

### a) Binding buffer screening

In a first round, several binding buffers at different concentrations were screened and the elution was performed with an elution buffer comprising 2 M NaCl in 20 mM Na-Phosphate at pH 7.5. Agarose gels were loaded with eluates according to **Table 1** below. Binding buffer salts listed in the table were used in conjunction with 20 mM sodium phosphate at pH 7.0 unless indicated otherwise.

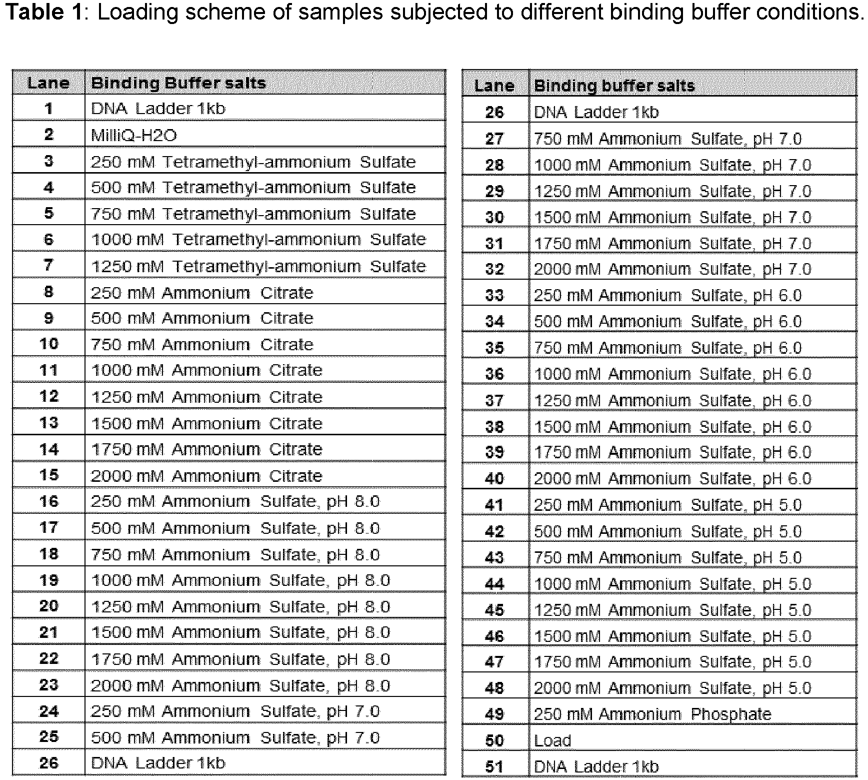

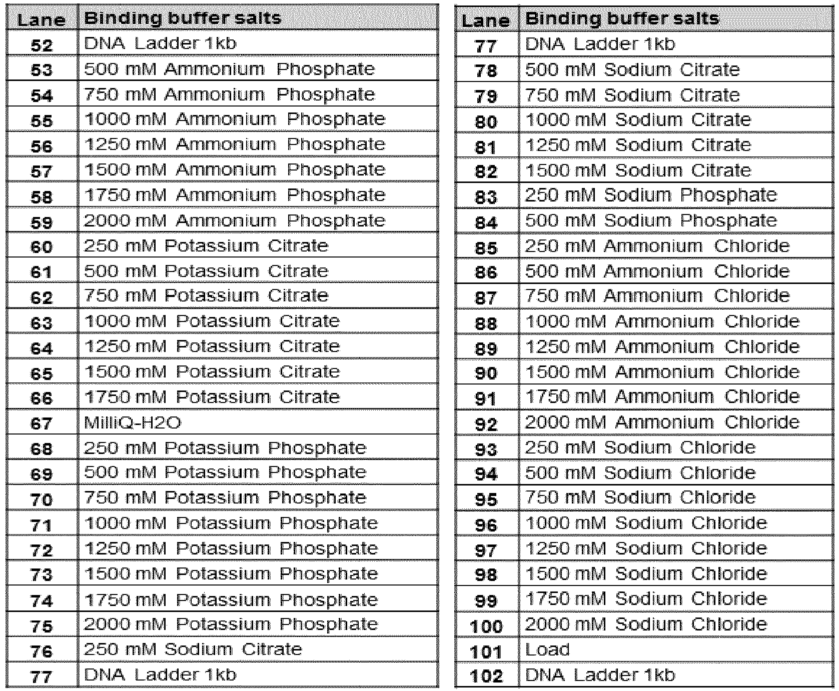

Results of the binding buffer screening effort are shown in **Figures 2A to 2D****,** wherein the upper and lower panel show the same gel after different exposure times (upper panel = shorter exposure time; lower panel = longer exposure time). The labeling on the right side of the gel depicts the bands of open circular (oc) and super coiled (sc) pUC19 plasmid DNA and RNA impurities within the agarose gel.

The results suggest that binding at high salt concentrations is feasible, particularly when using kosmotropic salts (e.g. ammonium citrate, ammonium sulfate, ammonium phosphate, potassium citrate, potassium phosphate, sodium citrate, sodium phosphate, etc.), and is essentially independent of the pH of the binding buffer. The results also show that, particularly at higher salt concentrations, binding of RNA is markedly reduced as compared to the load (cf., lanes 50 and 101) or does not bind at all (e.g., lanes 10 - 15).

### b) Elution buffer screening

In a second round, several elution buffers were screened at different concentrations and the binding of the sample mixture was accomplished with a binding buffer comprising 20 mM Na-Phosphate and 1.5 M Ammonium Sulfate at pH 7.0. Agarose gels were loaded with eluates according to **Table 2** below. Elution buffer salts listed in the table were used together with 20 mM Na-Phosphate at pH 7.0 unless indicated otherwise.

**Table 2: Loading scheme of samples subjected to different elution buffer conditions.**

| **Lane** | **Elution buffer salts** |
|---|---|
| **1** | DNA Ladder 1kb |
| **2** | 250 mM Sodium Acetate |
| **3** | 500 mM Sodium Acetate |
| **4** | 750 mM Sodium Acetate |
| **5** | 1000 mM Sodium Acetate |
| **6** | 1250 mM Sodium Acetate |
| **7** | 1500 mM Sodium Acetate |
| **8** | 1750 mM Sodium Acetate |
| **9** | 2000 mM Sodium Acetate |
| **10** | 250 mM Ammonium Chloride |
| **11** | 500 mM Ammonium Chloride |
| **12** | 750 mM Ammonium Chloride |
| **13** | 1000 mM Ammonium Chloride |
| **14** | 1250 mM Ammonium Chloride |
| **15** | 1500 mM Ammonium Chloride |
| **16** | 1750 mM Ammonium Chloride |
| **17** | 2000 mM Ammonium Chloride |
| **18** | 250 mM Potassium Chloride |
| **19** | 500 mM Potassium Chloride |
| **20** | 750 mM Potassium Chloride |
| **21** | 1000 mM Potassium Chloride |
| **22** | 1250 mM Potassium Chloride |
| **23** | 1500 mM Potassium Chloride |
| **24** | 1750 mM Potassium Chloride |
| **25** | 2000 mM Potassium Chloride |
| **26** | DNA Ladder 1kb |
| **26** | DNA Ladder 1kb |
| **27** | 250 mM Sodium Chloride |
| **28** | 500 mM Sodium Chloride |
| **29** | 750 mM Sodium Chloride |
| **30** | 1000 mM Sodium Chloride |
| **31** | 1250 mM Sodium Chloride |
| **32** | 1500 mM Sodium Chloride |
| **33** | 1750 mM Sodium Chloride |
| **34** | 2000 mM Sodium Chloride |
| **35** | 250 mM Magnesium Sulfate |
| **36** | 500 mM Magnesium Sulfate |
| **37** | 750 mM Magnesium Sulfate |
| **38** | 1000 mM Magnesium: Sulfate |
| **39** | 1250 mM Magnesium Sulfate |
| **40** | 1500 mM Magnesium Sulfate |
| **41** | 1750 mM Magnesium Sulfate |
| **42** | 2000 mM Magnesium Sulfate |
| **43** | 250 mM Magnesium Chloride |
| **44** | 500 mM Magnesium Chloride |
| **45** | 750 mM Magnesium Chloride |
| **46** | 1000 mM Magnesium Chloride |
| **47** | 1250 mM Magnesium Chloride |
| **48** | 1500 mM Magnesium Chloride |
| **49** | 1750 mM Magnesium Chloride |
| **50** | Load |
| **51** | DNA Ladder 1kb |
| **52** | DNA Ladder 1kb |
| **53** | 2000 mM Magnesium Chloride |
| **54** | 250 mM Magnesium Nitrate |
| **55** | 500 mM Magnesium Nitrate |
| **56** | 750 mM Magnesium Nitrate |
| **57** | 1000 mM Magnesium Nitrate |
| **58** | 1250 mM Magnesium Nitrate |
| **59** | 1500 mM Magnesium Nitrate |
| **60** | MilliQ-H2O |
| **61** | 20 mM Sodium Citrate, pH 2.0 |
| **62** | 20 mM Sodium Citrate, pH 2.5 |
| **63** | 20 mM Sodium Citrate, pH 3.0 |
| **64** | 20 mM Sodium Citrate, pH 3.5 |
| **65** | 20 mM Sodium Citrate, pH 4.0 |
| **66** | 20 mM Sodium Citrate, pH 4.5 |
| **67** | 20 mM Sodium Citrate, pH 5.0 |
| **68** | 20 mM Sodium Citrate, pH 5.5 |
| **69** | 50 mM Glycine, pH 2.0 |
| **70** | 50 mM Glycine, pH 2.5 |
| **71** | 50 mM Glycine, pH 3.0 |
| **72** | 20mM Sodium Acetate, pH 3.5 |
| **73** | 20mM Sodium Acetate, pH 4.0 |
| **74** | 20mM Sodium Acetate, pH 4.5 |
| **75** | 20mM Sodium Acetate, pH 5.0 |
| **76** | 50 mM Sodium Formate, pH 3.0 |
| **77** | DNA Ladder 1kb |

| **Lane** | **Elution butter salts** |
|---|---|
| **77** | DNA Ladder 1 kb |
| **78** | 50 mM Sodium Formate, pH 3.5 |
| **79** | 50 mM Sodium Formate, pH 4.0 |
| **80** | 50 mM Sodium Formate, pH 4.5 |
| **81** | 50 mM Na-Phosphate pH 5.5 |
| **82** | 50 mM Na-Phosphate, pH 60 |
| **83** | 50 mM Na-Phosphate, pH 6.5 |
| **84** | 50 mM Na-Phosphate, pH 7.0 |
| **85** | 1% Tween 20 |
| **86** | 0.1% Tween 20 |
| **87** | 1 % CTAB |
| **88** | 0.1% CTAB |
| **89** | 1% SDS |
| **90** | 0.1% SDS |
| **91** | 1% SDS |
| **92** | 0.1% SDS |
| **93** | 30% EtOH |
| **94** | 30% IPA |
| **95** | 15% DMSO |
| **96** | 7 M urea |
| **97** | 6 M GuHCl |
| **98** | 0.8 M Arqinine |
| **99** | 0.8 M Lysine |
| **100** | 0.25 M Histidine |
| **101** | Load |
| **102** | DNA Ladder 1 kb |

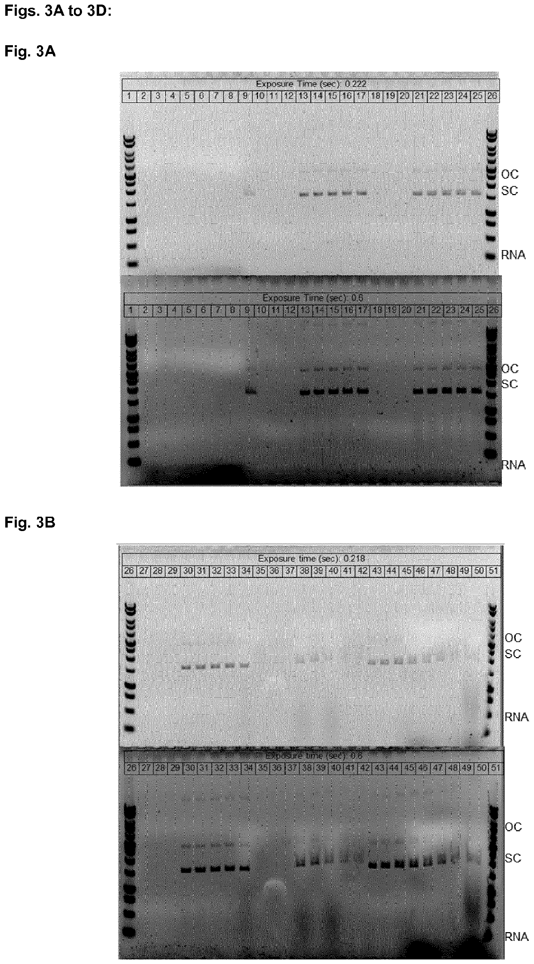

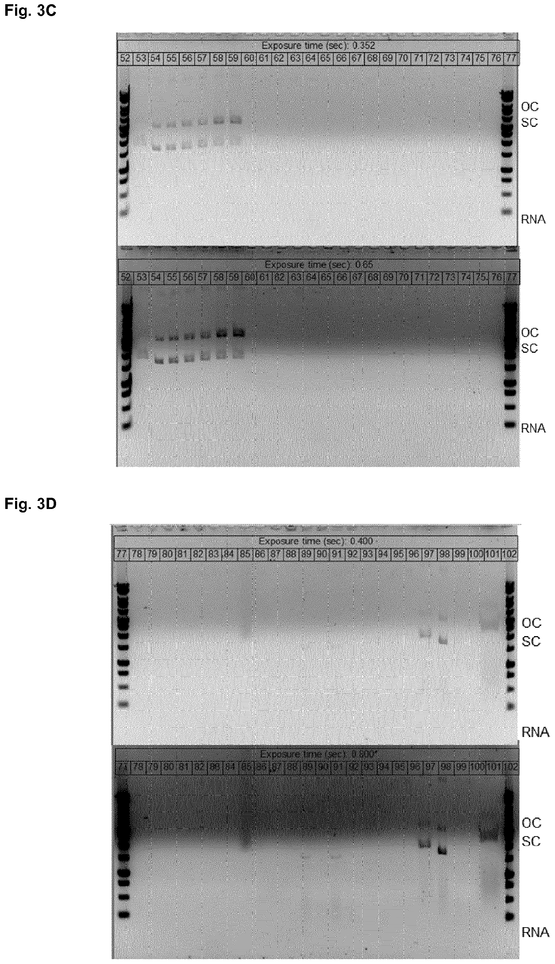

Results of the elution buffer screening effort are shown in Figure 3A to 3D, wherein the upper and lower panel show the same gel after different exposure times (upper panel = shorter exposure time; lower panel = longer exposure time). The labeling on the right side of the gel depicts the bands of open circular (oc) and super coiled (sc) pUC19 plasmid DNA and RNA impurities within the agarose gel.

The results show that plasmid DNA can be eluted from the AEX resin when using chaotropic salts (e.g. ammonium chloride, potassium chloride, sodium chloride, magnesium sulfate, magnesium chloride, magnesium nitrate, etc.) even in cases where the conductivity of the elution buffer is significantly lower than the conductivity of the binding buffer. Moreover, most of the tested elution buffers elute sc pDNA better than oc pDNA, and do not elute RNA. However, it was observed that certain chaotropic salts, e.g. magnesium nitrate, preferentially eluted oc pDNA over sc pDNA (cf. Fig. 3C).

### Example 2: pUC19 purification using Sartobind Q and magnesium sulfate for the elution with a phosphate buffer system

### a) Purification procedure

All buffers used throughout the process were filtered through 0.2 µm filters. E. coli cells harboring the plasmid pUC19 were grown and lysed as described in Example 1.

The 150 mL solution containing pUC19 was conditioned with 150 mL conditioning buffer (40 mM Na-Phosphate, pH 7.0, 3 M Ammonium Sulfate). The chromatography unit was equilibrated with 20 column volumes (CVs) of 20 mM Na-Phosphate, 1.5 M Ammonium Sulfate, pH 7.0 at a flow rate of 4 CVs/min. The conditioned load was then applied to the anion exchange unit. The unit was washed by applying 20 CVs equilibration buffer followed by a second wash of 20 CVs of wash buffer 2 (20 mM Na-phosphate, 0.5 M magnesium sulfate, pH 7.0) which was found to remove RNA impurities. Plasmid DNA was then eluted by applying a gradient from wash buffer 2 to elution buffer (20 mM Na-Phosphate, 1.0 M Magnesium Sulfate, pH 7.0) in 25 CVs. Finally, the chromatography unit was stripped by applying 30 CVs of 4 M NaCl.

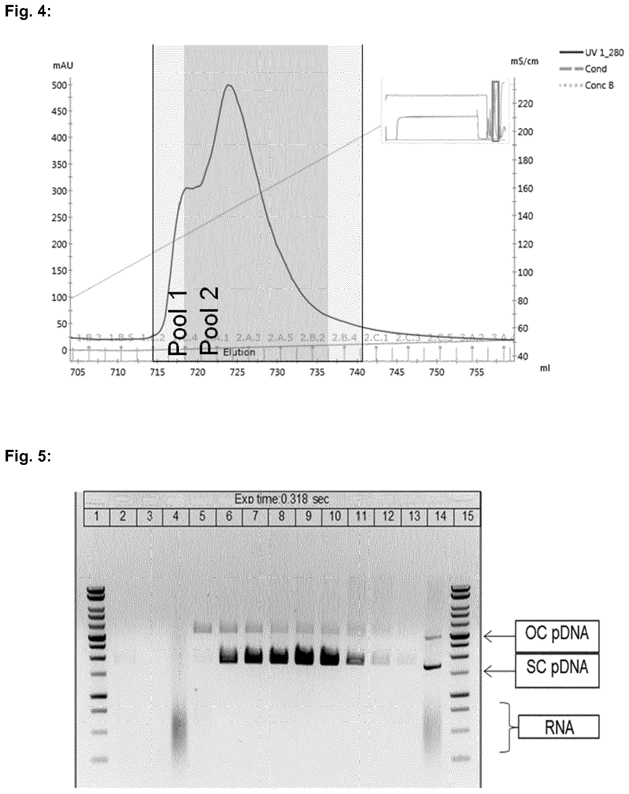

A representative chromatography profile of the anion exchange unit (Sartobind Q) is shown in **Figure 4** with a zoom-in on the elution gradient. Two pools were established: Pool 1 comprising fractions 1C2 to 2B4 (light grey box) and Pool 2 comprising fractions 1C4 to 2B2 (dark grey box).

### b) Assessment of residual impurities

Fractions from the AEX chromatography step with Sartobind Q were analyzed by agarose gel electrophoresis (cf. **Table 3** below for the loading scheme of the agarose gel) and the results are shown in **Figure 5****.**

**Table 3: Loading scheme of fractions from the anion exchange chromatography step (Sartobind Q) as analyzed by agarose gel electrophoresis.**

| **Well no.** | **Sample** |
|---|---|
| 1 | 1 kb ladder |
| 2 | Load |
| 3 | Load FT |
| 4 | Wash 2 |
| 5 | Fr. 1A2 |
| 6 | Fr. 1C3 |
| 7 | Fr. 1C4 |
| 8 | Fr. 1C5 |
| 9 | Fr. 2A1 |
| 10 | Fr. 2A2 |
| 11 | Fr. 2A4 |
| 12 | Fr. 2B1 |
| 13 | Fr. 2B3 |
| 14 | Strip |
| 15 | 1 kb ladder |

High amounts were loaded on the agarose gel to visualize impurities. Figure 5 shows that RNA was largely removed during the second wash step (see, e.g., lane 4; washing buffer: 50 mM Tris-base/TAPS ([tris(hydroxymethyl)methylamino]propanesulfonic acid), 0.5 M magnesium sulfate, pH 7.0). For the gradient elution under the chosen conditions, it was observed that oc pDNA had a lower retention time than SC pDNA (see again Figure 5).

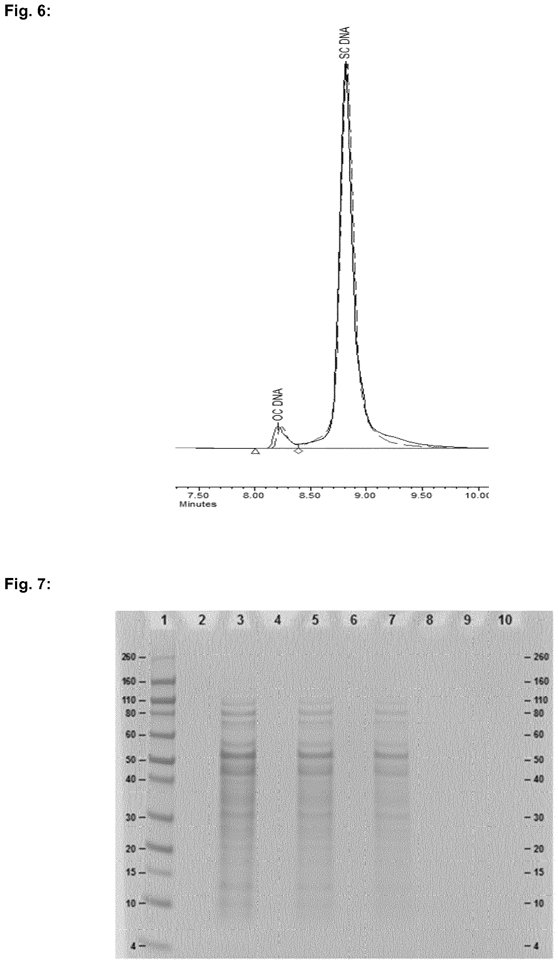

Fractions from the AEX chromatography step (Sartobind Q) were also analyzed by HPLC. AEX-HPLC traces from the Sartobind Q elution pool 1 (cf. **Figure 4****;** dashed line) and a standard (solid line) are shown in **Figure 6** and the results are summarized in **Table 4** below. To measure residual RNA within the load and elution pool 1, the pDNA was transferred to water by performing a tangential flow filtration step (Pellicon 3 cassette, 30 kDa, C screen, Ultracel membrane, Cat#: P3C030C00, Merck Millipore). Since pool 2 was a subset of pool 1, residual RNA was not measured for pool 2.

**Table 4: Analytical results for the AEX unit operation.**

| **Sample** | **UV Absorption** | | **AEX HPLC** | **RNA** |
|---|---|---|---|---|
| Product description | A260 (AU) | DNA titer (µg/mL) | Sum OC and L-Form (% *impurities)* | Residual RNA (µg/mL) |
| Load | 1.2 | 60 | n/a | 59.1 |
| Pool 1 | 6.0 | 302 | 4 | < 1.6 |
| Pool 2 | 8.5 | 425 | 3 | < LoQ |

The results summarized in **Table 4** above demonstrate that RNA was removed quantitatively. The oc pDNA and linear pDNA was reduced to low levels of 4% and 3% for Pool 1 and Pool 2, respectively. Taken together, the gradient elution allowed separation of RNA, as well as open-circular plasmid DNA (oc pDNA) from super-coiled plasmid DNA (sc pDNA). SDS-PAGE analysis **(****Figure 7****)** confirmed that proteinaceous impurities were likewise removed quantitatively by the AEX chromatography step (cf. Table 5 for the loading scheme of the SDS-PAGE gel).

**Table 5: Loading scheme of samples as analyzed by SDS-PAGE.**

| **Lane** | **Sample** |
|---|---|
| 1 | Ladder |
| 2 | Blank |
| 3 | Load non-diluted |
| 4 | Blank |
| 5 | Load 1:1 dilution |
| 6 | Blank |
| 7 | Load 1:2 dilution |
| 8 | Elution pool non-diluted |
| 9 | Elution 1:1 dilution |
| 10 | Elution 1:2 dilution |

### Example 3: Improved primary recovery process and pDNA pUC19 purification using Sartobind Q and magnesium sulfate for the elution with a Tris buffer system

E. coli cells harboring the plasmid pUC19 were grown and lysed as described in more detail below. All buffers used throughout the process were filtered through 0.2 µm filters. A schematic representation of the primary recovery process including the AEX chromatography step is depicted in **Figure 8****.**

For the primary recovery, a 76.4 g pellet of E. coli harboring the plasmid pUC19 was suspended with 764 mL suspension buffer (50 mM Tris, 10 mM EDTA) for 1 hour. Addition of 764 mL lysis buffer (1 % SDS, 0.2 M NaOH) started lysis of the cells. The lysis process was stopped after 4 minutes by adding 198 mL neutralization buffer (3 M Potassium Acetate, 2 M Acetic acid, chilled at 5 °C). It was found that 0.26 volumes are sufficient for neutralization.

1800 mL of the neutralized solution was conditioned in a tulip-shaped tank by adding 1800 mL of conditioning buffer (200 mM Tris-base/TAPS, 3 M Ammonium Sulfate pH 7.0) to increase the density of the solution. The tulip-shaped tank facilitates removal of insoluble particles and thus improves the filtration behavior of the conditioned solution when applied to a depth filter (Clarisolve; Cat#: CS40MS01L3; 40 µm, Merck Millipore). The pH value of the filtrate was adjusted to 7.0 by adding 194 mL 1 M NaOH.

The chromatography unit was equilibrated with 20 column volumes (CVs) of 50 mM Tris-base/TAPS, 1.5 M ammonium sulfate, pH 7.0 at a flow rate of 4 CVs/min. The depth filtrate was then applied to the AEX unit (Sartobind Q; Cat# 96IEXQ42EUC11-A; 3 mL bed volume; Sartorius). Binding of plasmid DNA was performed at a concentration of 1.5 M ammonium sulfate. The load flow-through showed high absorbance values indicating that proteins and a significant amount of RNA species did not bind to the AEX material under these loading conditions. The unit was then washed by applying 20 CVs equilibration buffer followed by a second wash with 20 CVs of wash buffer (50 mM Tris-Base/TAPS, 0.5 M magnesium sulfate, pH 7.0) to remove RNA impurities. Plasmid DNA was eluted by applying a gradient from wash to elution buffer (50 mM Tris-Base/TAPS, 1.0 M magnesium sulfate, pH 7.0) in 25 CVs. The gradient elution allowed separation of mainly two species, wherein the first peak contained oc pDNA and the second peak contained sc pDNA. The chromatography unit was finally stripped by applying 30 CVs of 4 M NaCl. A representative chromatography profile of the anion exchange unit (Sartobind Q) is shown in **Figure 9****.**

## Claims

1. A method for isolating or purifying a plasmid DNA ("pDNA") of interest from a mixture, the method comprising:
contacting the mixture with an anion exchange material in the presence of a solution comprising a kosmotropic salt at a concentration that allows selective binding of the plasmid DNA (pDNA) of interest to the anion exchange material;
wherein the kosmotropic salt is selected from the group consisting of: ammonium sulfate, ammonium citrate, ammonium phosphate, potassium phosphate, sodium citrate, sodium phosphate, and mixtures thereof;
wherein the concentration of the kosmotropic salt is greater than about 0.5 M; and
eluting the nucleic acid molecule of interest with an eluent comprising a chaotropic salt at a concentration that provides an eluant containing the purified nucleic acid molecule;
optionally wherein
(i) the fraction(s) comprising said nucleic acid molecule in the eluant is/are collected; *and*/*or*
(ii) said nucleic acid molecule of interest is isolated from any one or all of the collected fractions; *and*/*or*
(iii) the method is conducted in the absence of organic solvents, detergents, glycols, hexamine cobalt, spermidine, *and*/*or* polyvinylpyrrolidone.

2. The method of claim 1, wherein
(i) the plasmid DNA is super-coiled plasmid DNA (sc pDNA); *and*/*or*
(ii) the plasmid DNA comprises mammalian DNA, bacterial DNA, non-coding DNA, or viral DNA; optionally wherein the plasmid DNA comprises DNA capable of expressing a polypeptide of interest; *and*/*or*
(iii) the mixture comprises super-coiled plasmid DNA, open-circular plasmid DNA, linear plasmid DNA, genomic DNA, RNA, lipopolysaccharides, endotoxins, proteins, or any combination thereof.

3. The method of claim 1 or claim 2, wherein the purity of the purified *and*/*or* isolated plasmid DNA is at least 90%, or at least 95%, or at least 98%.

4. The method of any one of claims 1 to 3, wherein
(i) the concentration of the kosmotropic salt is about 0.8 M to about 4.0 M; *and*/*or*
(ii) the concentration of the chaotropic salt that provides an eluant containing the purified nucleic acid molecule is about 0.25 M to about 4.0 M; *and*/*or*
(iii) the elution step comprises a gradient elution by varying the concentration of the chaotropic salt in the eluent; optionally wherein the concentration of the chaotropic salt in the eluent is increased.

5. The method of any one of claims 2 to 4, wherein the elution step includes fractionation of super-coiled plasmid DNA from other plasmid DNA forms such as linear plasmid DNA and open-circular plasmid DNA, and, optionally, other nucleic acid molecules.

6. The method of any one of claims 1 to 5, wherein
(i) the kosmotropic salt is ammonium sulfate; optionally wherein the concentration of ammonium sulfate is about 0.5 M to about 2.0M; *and*/*or*
(ii) wherein the chaotropic salt is selected from the group consisting of: ammonium chloride, potassium chloride, sodium chloride, magnesium sulfate, magnesium chloride, magnesium nitrate, guanidinium hydrochloride, and mixtures thereof, optionally wherein the chaotropic salt is magnesium sulfate; further optionally wherein the concentration of magnesium sulfate is about 0.5M to about 1.0M; *and*/*or*
(iii) the solution comprising the kosmotropic salt has a pH in the range of about 5.0 to about 10.0, or of about 5.0 to about 8.0, or about 6.0 to about 8.0, or about pH 7.

7. The method of any one of claims 1 to 6, wherein the anion exchange material is selected from an anion exchange membrane, an ion exchange resin, a three-dimensional microporous hydrogel structure, a packed bed of superporous beads, macroporous beads, a monolith, agarose beads, cross-linked agarose, silica beads, large pore gels, methacrylate-based beads, polysterene-based beads, cellulose-based beads, dextran-based beads, bisacrylamide-based beads, polyvinylether-based beads, ceramic-based beads, or polymer-based beads;
optionally wherein the anion exchange material is
(i) an anion exchange membrane; optionally wherein the anion exchange membrane has an average pore size of about 3.0µm to about 5.0µm; preferably wherein the average pore size is about 3.0µm; or
(ii) an ion exchange resin, optionally wherein the ion exchange resin has an average particle diameter from about 30 µm to about 300µm.

8. The method of any one of claims 1 to 7, wherein the method further comprises separating solid components from the mixture comprising the nucleic acid molecule of interest *prior to* contacting the mixture with an anion exchange material in the presence of the solution comprising the salt at a concentration that allows selective binding of the nucleic acid molecule to the anion exchange material,
optionally wherein said removal of solid components is achieved by filtration or phase separation.

9. The method of claim 8, wherein the removal of solid components is achieved via two-phase separation,
optionally wherein
(i) a buffer is added to increase the density of the mixture to be greater than about 1.1 kg/L, optionally wherein the buffer comprises a kosmotropic salt, preferably wherein the kosmotropic salt is ammonium sulfate; *and*/*or*
(ii) wherein the lower phase of the two-phase mixture having a higher density than the top phase is subjected to a depth filtration.

10. The method of any one of claims 1 to 9, wherein the method further comprises the step of
(i) precipitating the nucleic acid molecule from the eluant; *and*/*or*
(ii) filtering the eluant by tangential-flow filtration to isolate the nucleic acid molecule; optionally wherein the average pore size of the filtration membrane used in the tangential-flow filtration step is ≤ 0.2 µm; *and*/*or*
(iii) lyophilization of the purified nucleic acid molecule of interest, optionally wherein the lyophilization is carried out in the presence of a carbohydrate; *and*/*or*
(iv) collecting a flow-through following the step of contacting the mixture with an anion exchange material in the presence of the solution comprising the salt at a concentration that allows selective binding of the nucleic acid molecule to the anion exchange material; optionally wherein the flow-through comprises RNA, genomic DNA, proteins, cellular fractions, or combinations thereof, preferably wherein the flow-through comprises RNA; *and*/*or*
(v) washing the anion exchange material with a washing buffer solution prior to the elution of the nucleic acid molecule of interest; optionally wherein the washing buffer solution comprises a chaotropic salt at a concentration lower than the concentration required for the elution of the nucleic acid molecule of interest, wherein the chaotropic salt is preferably selected from the group consisting of: ammonium chloride, potassium chloride, sodium chloride, magnesium sulfate, magnesium chloride, magnesium nitrate, and mixtures thereof.

11. The method of any one of claims 1 to 10, wherein the method further comprises a further purification step following the step of eluting and optionally isolating the nucleic acid molecule;
optionally wherein the second purification step comprises use of a thiophilic aromatic adsorption chromatography medium having a selectivity that allows super-coiled plasmid DNA to be separated from open circular *and*/*or* linear DNA.

12. The method of any one of claims 1 to 11, wherein the method further comprises the step of
(i) using the nucleic acid to express a polypeptide of interest; preferably wherein the nucleic acid is plasmid DNA; or
(ii) using the nucleic acid to produce and harvest a polypeptide of interest; preferably wherein the nucleic acid is plasmid DNA; optionally wherein the method further comprises the step of formulating the polypeptide of interest into a pharmaceutical composition.

13. The method of any one of claims 2 to 11, wherein the method further comprises the step of using the purified plasmid DNA for the production of RNA.

14. The method of any one of claims 1 to 13, wherein the method includes the steps of cell harvesting and washing, cell lysis, neutralization, and flocculate removal prior to contacting the resulting mixture comprising the nucleic acid molecule of interest with the anion exchange material.

15. The method of any one of claims 1 to 14, wherein the method does not comprise a CaCl₂ precipitation step to remove RNA.

## Patentansprüche

1. Verfahren zur Isolierung oder Reinigung einer Plasmid-DNA ("pDNA") von Interesse aus einem Gemisch, wobei das Verfahren umfasst:
Inkontaktbringen der Mischung mit einem Anionenaustauschermaterial in Gegenwart einer Lösung, die ein kosmotropes Salz in einer Konzentration umfasst, die eine selektive Bindung der interessierenden Plasmid-DNA (pDNA) an das Anionenaustauschermaterial ermöglicht,
wobei das kosmotrope Salz ausgewählt ist aus der Gruppe bestehend aus:
Ammoniumsulfat, Ammoniumcitrat, Ammoniumphosphat, Kaliumphosphat, Natriumcitrat, Natriumphosphat und Mischungen davon,
wobei die Konzentration des kosmotropen Salzes größer als etwa 0,5 M ist; und
Eluieren des Nukleinsäuremoleküls von Interesse mit einem Elutionsmittel, das ein chaotropes Salz in einer Konzentration umfasst, die ein Elutionsmittel liefert, das das gereinigte Nukleinsäuremolekül enthält;
wobei wahlweise
(i) die Fraktion(en), die das Nukleinsäuremolekül in dem Elutionsmittel enthält/enthalten, gesammelt wird/werden; *und*/*oder*
(ii) das Nukleinsäuremolekül von Interesse aus einer oder allen der gesammelten Fraktionen isoliert wird; *und*/*oder*
(iii) das Verfahren in Abwesenheit von organischen Lösungsmitteln, Detergenzien, Glykolen, Hexaminkobalt, Spermidin, *und*/*oder* Polyvinylpyrrolidon durchgeführt wird.

2. Verfahren nach Anspruch 1, wobei
(i) die Plasmid-DNA supergewickelte Plasmid-DNA (sc pDNA) ist; *und*/*oder*
(ii) die Plasmid-DNA Säugetier-DNA, bakterielle DNA, nicht-kodierende DNA oder virale DNA umfasst; wobei die Plasmid-DNA gegebenenfalls DNA umfasst, die ein Polypeptid von Interesse exprimieren kann; *und*/*oder*
(iii) das Gemisch super-gewickelte Plasmid-DNA, offen-zirkuläre Plasmid-DNA, lineare Plasmid-DNA, genomische DNA, RNA, Lipopolysaccharide, Endotoxine, Proteine oder irgendeine Kombination davon umfasst.

3. Verfahren nach Anspruch 1 oder Anspruch 2, wobei die Reinheit der gereinigten und/oder isolierten Plasmid-DNA mindestens 90 % oder mindestens 95 % oder mindestens 98 % beträgt.

4. Das Verfahren nach einem der Ansprüche 1 bis 3, wobei
(i) die Konzentration des kosmotropen Salzes etwa 0,8 M bis etwa 4,0 M beträgt; *und*/*oder*
(ii) die Konzentration des chaotropen Salzes, die ein Elutionsmittel liefert, das das gereinigte Nukleinsäuremolekül enthält, etwa 0,25 M bis etwa 4,0 M beträgt; *und*/*oder*
(iii) der Elutionsschritt eine Gradientenelution durch Variieren der Konzentration des chaotropen Salzes im Elutionsmittel umfasst; wobei die Konzentration des chaotropen Salzes im Elutionsmittel gegebenenfalls erhöht wird.

5. Verfahren nach einem der Ansprüche 2 bis 4, wobei der Elutionsschritt die Fraktionierung von super-gewickelter Plasmid-DNA von anderen Plasmid-DNA-Formen, wie linearer Plasmid-DNA und offen-zirkulärer Plasmid-DNA, und gegebenenfalls anderen Nukleinsäuremolekülen umfasst.

6. Das Verfahren nach einem der Ansprüche 1 bis 5, wobei
(i) das kosmotrope Salz Ammoniumsulfat ist; optional, wobei die Konzentration von Ammoniumsulfat etwa 0,5 M bis etwa 2,0 M beträgt; *und*/*oder*
(ii) wobei das chaotrope Salz ausgewählt ist aus der Gruppe bestehend aus: Ammoniumchlorid, Kaliumchlorid, Natriumchlorid, Magnesiumsulfat, Magnesiumchlorid, Magnesiumnitrat, Guanidiniumhydrochlorid und Mischungen davon, wobei das chaotrope Salz gegebenenfalls Magnesiumsulfat ist; wobei ferner gegebenenfalls die Konzentration von Magnesiumsulfat etwa 0,5 M bis etwa 1,0 M beträgt; *und*/*oder*
(iii) die Lösung, die das kosmotrope Salz enthält, einen pH-Wert im Bereich von etwa 5,0 bis etwa 10,0 oder von etwa 5,0 bis etwa 8,0 oder von etwa 6,0 bis etwa 8,0 oder etwa pH 7 aufweist.

7. Verfahren nach einem der Ansprüche 1 bis 6, wobei das Anionenaustauschermaterial ausgewählt ist aus einer Anionenaustauschermembran, einem lonenaustauscherharz, einer dreidimensionalen mikroporösen Hydrogelstruktur, einem gepackten Bett aus superporösen Perlen, makroporösen Perlen, einem Monolithen, Agarosekügelchen, vernetzte Agarose, Kieselsäurekügelchen, großporige Gele, Kügelchen auf Methacrylatbasis, Kügelchen auf Polystyrolbasis, Kügelchen auf Cellulosebasis, Kügelchen auf Dextranbasis, Kügelchen auf Bisacrylamidbasis, Kügelchen auf Polyvinyletherbasis, Kügelchen auf Keramikbasis oder Kügelchen auf Polymerbasis; wobei es sich bei dem Anionenaustauschmaterial gegebenenfalls um
(i) eine Anionenaustauschmembran ist; wobei die Anionenaustauschmembran gegebenenfalls eine durchschnittliche Porengröße von etwa 3,0 µm bis etwa 5,0 µm aufweist; vorzugsweise wobei die durchschnittliche Porengröße etwa 3,0 µm beträgt; oder
(ii) ein lonenaustauscherharz ist, wobei das lonenaustauscherharz gegebenenfalls einen durchschnittlichen Teilchendurchmesser von etwa 30 µm bis etwa 300 µm aufweist.

8. Verfahren nach einem der Ansprüche 1 bis 7, wobei das Verfahren ferner die Abtrennung fester Komponenten aus dem Gemisch, das das interessierende Nukleinsäuremolekül umfasst, umfasst, *bevor* das Gemisch mit einem Anionenaustauschermaterial in Gegenwart der Lösung, die das Salz umfasst, in einer Konzentration in Kontakt gebracht wird, die eine selektive Bindung des Nukleinsäuremoleküls an das Anionenaustauschermaterial ermöglicht,
wobei die Entfernung fester Komponenten gegebenenfalls durch Filtration oder Phasentrennung erreicht wird.

9. Verfahren nach Anspruch 8, wobei die Entfernung der festen Bestandteile durch Zweiphasentrennung erreicht wird,
wobei gegebenenfalls
(i) ein Puffer zugegeben wird, um die Dichte der Mischung auf mehr als etwa 1,1 kg/L zu erhöhen, wobei der Puffer gegebenenfalls ein kosmotropes Salz umfasst, vorzugsweise wobei das kosmotrope Salz Ammoniumsulfat ist; *und*/*oder*
(ii) wobei die untere Phase der zweiphasigen Mischung, die eine höhere Dichte als die obere Phase aufweist, einer Tiefenfiltration unterzogen wird.

10. Verfahren nach einem der Ansprüche 1 bis 9, wobei das Verfahren ferner den Schritt umfasst:
(i) Ausfällen des Nukleinsäuremoleküls aus dem Eluat; *und*/*oder*
(ii) Filtrieren des Eluats durch Tangentialflussfiltration, um das Nukleinsäuremolekül zu isolieren; wobei optional die durchschnittliche Porengröße der in dem Tangentialflussfiltrationsschritt verwendeten Filtrationsmembran ≤ 0,2 µm ist; *und*/*oder*
(iii) Lyophilisierung des gereinigten Nukleinsäuremoleküls von Interesse, wobei die Lyophilisierung gegebenenfalls in Gegenwart eines Kohlenhydrats durchgeführt wird; *und*/*oder*
(iv) Sammeln eines Durchflusses nach dem Schritt des In-Kontakt-Bringens der Mischung mit einem Anionenaustauschermaterial in Gegenwart der Lösung, die das Salz in einer Konzentration umfasst, die eine selektive Bindung des Nukleinsäuremoleküls an das Anionenaustauschermaterial ermöglicht; wobei der Durchfluss gegebenenfalls RNA, genomische DNA, Proteine, zelluläre Fraktionen oder Kombinationen davon umfasst, vorzugsweise wobei der Durchfluss RNA umfasst; *und*/*oder*
(v) Waschen des Anionenaustauschermaterials mit einer Waschpufferlösung vor der Elution des interessierenden Nukleinsäuremoleküls; wobei die Waschpufferlösung gegebenenfalls ein chaotropes Salz in einer Konzentration umfasst, die niedriger ist als die Konzentration, die für die Elution des interessierenden Nukleinsäuremoleküls erforderlich ist, wobei das chaotrope Salz vorzugsweise ausgewählt ist aus der Gruppe bestehend aus: Ammoniumchlorid, Kaliumchlorid, Natriumchlorid, Magnesiumsulfat, Magnesiumchlorid, Magnesiumnitrat und Mischungen davon.

11. Verfahren nach einem der Ansprüche 1 bis 10, wobei das Verfahren ferner einen weiteren Reinigungsschritt im Anschluss an den Schritt des Eluierens und gegebenenfalls des Isolierens des Nukleinsäuremoleküls umfasst;
wobei der zweite Reinigungsschritt gegebenenfalls die Verwendung eines thiophilen aromatischen Adsorptionschromatographiemediums mit einer Selektivität umfasst, die es ermöglicht, super-gewickelte Plasmid-DNA von offener zirkulärer *und*/*oder* linearer DNA zu trennen.

12. Verfahren nach einem der Ansprüche 1 bis 11, wobei das Verfahren ferner den folgenden Schritt umfasst:
(i) Verwendung der Nukleinsäure zur Expression eines Polypeptids von Interesse, wobei die Nukleinsäure vorzugsweise Plasmid-DNA ist, oder
(ii) Verwendung der Nukleinsäure zur Herstellung und Gewinnung eines Polypeptids von Interesse; vorzugsweise wobei es sich bei der Nukleinsäure um Plasmid-DNA handelt; wobei das Verfahren gegebenenfalls weiterhin den Schritt der Formulierung des Polypeptids von Interesse in einer pharmazeutischen Zusammensetzung umfasst.

13. Verfahren nach einem der Ansprüche 2 bis 11, wobei das Verfahren ferner den Schritt der Verwendung der gereinigten Plasmid-DNA für die Herstellung von RNA umfasst.

14. Verfahren nach einem der Ansprüche 1 bis 13, wobei das Verfahren die Schritte der Zellernte und des Waschens, der Zelllyse, der Neutralisierung und der Enfernung von Ausflockungen einschließt, bevor die resultierende Mischung, die das interessierende Nukleinsäuremolekül enthält, mit dem Anionenaustauschermaterial in Kontakt gebracht wird.

15. Verfahren nach einem der Ansprüche 1 bis 14, wobei das Verfahren keinen CaCl₂ - Fällungsschritt zur Entfernung von RNA umfasst.

## Revendications

1. Procédé pour isoler ou purifier un ADN plasmidique ("ADNp") d'intérêt à partir d'un mélange, le procédé comprenant :
la mise en contact du mélange avec un matériau échangeur d'anions en présence d'une solution comprenant un sel kosmotrope à une concentration qui permet la liaison sélective de l'ADN plasmidique (ADNp) d'intérêt au matériau échangeur d'anions ;
dans lequel le sel kosmotrope est choisi dans le groupe constitué par : le sulfate d'ammonium, le citrate d'ammonium, le phosphate d'ammonium, le phosphate de potassium, le citrate de sodium, le phosphate de sodium et leurs mélanges ;
dans lequel la concentration du sel kosmotrope est supérieure à environ 0,5 M ; et
élution de la molécule d'acide nucléique d'intérêt avec un éluant comprenant un sel chaotropique à une concentration qui fournit un éluant contenant la molécule d'acide nucléique purifiée ;
éventuellement dans laquelle
(i) la ou les fraction(s) comprenant ladite molécule d'acide nucléique dans l'éluant est/sont collectée(s); et/ou
(ii) ladite molécule d'acide nucléique d'intérêt est isolée de l'une quelconque ou de toutes les fractions collectées; et/ou
(iii) la méthode est conduite en l'absence de solvants organiques, de détergents, de glycols, d'hexamine-cobalt, de spermidine et/ou de polyvinylpyrrolidone.

2. Procédé de la revendication 1, dans lequel
(i) l'ADN plasmidique est un ADN plasmidique supercoiffé (sc pDNA) ; *et*/*ou*
(ii) l'ADN plasmidique comprend de l'ADN de mammifère, de l'ADN bactérien, de l'ADN non codant ou de l'ADN viral ; éventuellement, l'ADN plasmidique comprend de l'ADN capable d'exprimer un polypeptide d'intérêt ; *et*/*ou*
(iii) le mélange comprend de l'ADN plasmidique super enroulé, de l'ADN plasmidique en circuit ouvert, de l'ADN plasmidique linéaire, de l'ADN génomique, de l'ARN, des lipopolysaccharides, des endotoxines, des protéines, ou toute combinaison de ceux-ci.

3. Procédé de la revendication 1 ou de la revendication 2, dans lequel la pureté de l'ADN plasmidique purifié et/ou isolé est d'au moins 90 %, ou d'au moins 95 %, ou d'au moins 98 %.

4. Procédé de l'une quelconque des revendications 1 à 3, dans lequel
(i) la concentration du sel kosmotrope est d'environ 0,8 M à environ 4,0 M ; *et*/*ou*
(ii) la concentration du sel chaotropique qui fournit un éluant contenant la molécule d'acide nucléique purifiée est d'environ 0,25 M à environ 4,0 M ; *et*/*ou*
(iii) l'étape d'élution comprend une élution à gradient en faisant varier la concentration du sel chaotropique dans l'éluant ; éventuellement dans laquelle la concentration du sel chaotropique dans l'éluant est augmentée.

5. Procédé de l'une quelconque des revendications 2 à 4, dans lequel l'étape d'élution comprend le fractionnement de l'ADN plasmidique superenroulé à partir d'autres formes d'ADN plasmidique telles que l'ADN plasmidique linéaire et l'ADN plasmidique à circuit ouvert, et, éventuellement, d'autres molécules d'acide nucléique.

6. Procédé de l'une quelconque des revendications 1 à 5, dans lequel
(i) le sel kosmotrope est du sulfate d'ammonium ; éventuellement dans lequel la concentration de sulfate d'ammonium est d'environ 0,5 M à environ 2,0 M ; *et*/*ou*
(ii) dans laquelle le sel chaotrope est choisi dans le groupe constitué par : le chlorure d'ammonium, le chlorure de potassium, le chlorure de sodium, le sulfate de magnésium, le chlorure de magnésium, le nitrate de magnésium, le chlorhydrate de guanidinium, et leurs mélanges, éventuellement dans laquelle le sel chaotrope est le sulfate de magnésium ; en outre, éventuellement dans laquelle la concentration de sulfate de magnésium est d'environ 0,5 M à environ 1,0 M ; *et*/*ou*
(iii) la solution comprenant le sel kosmotrope a un pH dans la gamme d'environ 5,0 à environ 10,0, ou d'environ 5,0 à environ 8,0, ou d'environ 6,0 à environ 8,0, ou d'environ pH 7.

7. Procédé de l'une quelconque des revendications 1 à 6, dans lequel le matériau échangeur d'anions est choisi parmi une membrane échangeuse d'anions, une résine échangeuse d'ions, une structure tridimensionnelle d'hydrogel microporeux, un lit tassé de billes superporeuses, des billes macroporeuses, un monolithe, des billes d'agarose, de l'agarose réticulé, des billes de silice, des gels à larges pores, des billes à base de méthacrylate, des billes à base de polysterène, des billes à base de cellulose, des billes à base de dextrane, des billes à base de bisacrylamide, des billes à base de polyvinyléther, des billes à base de céramique ou des billes à base de polymère ;
éventuellement dans lequel le matériau échangeur d'anions est
(i) une membrane échangeuse d'anions ; éventuellement dans laquelle la membrane échangeuse d'anions a une taille de pore moyenne d'environ 3,0 µm à environ 5,0 µm ; de préférence dans laquelle la taille de pore moyenne est d'environ 3,0 µm ; ou
(ii) une résine échangeuse d'ions, éventuellement dans laquelle la résine échangeuse d'ions a un diamètre de particule moyen d'environ 30 µm à environ 300 µm.

8. Procédé de l'une quelconque des revendications 1 à 7, dans lequel le procédé comprend en outre la séparation des composants solides du mélange comprenant la molécule d'acide nucléique d'intérêt *avant la* mise en contact du mélange avec un matériau échangeur d'anions en présence de la solution comprenant le sel à une concentration qui permet la liaison sélective de la molécule d'acide nucléique au matériau échangeur d'anions,
éventuellement dans lequel ladite élimination des composants solides est réalisée par filtration ou séparation de phases.

9. Procédé selon la revendication 8, dans lequel l'élimination des composants solides est réalisée par une séparation à deux phases,
éventuellement dans lequel
(i) un tampon est ajouté pour augmenter la densité du mélange pour qu'elle soit supérieure à environ 1,1 kg/L, éventuellement dans lequel le tampon comprend un sel kosmotrope, de préférence dans lequel le sel kosmotrope est du sulfate d'ammonium ; *et*/*ou*
(ii) dans lequel la phase inférieure du mélange à deux phases ayant une densité plus élevée que la phase supérieure est soumise à une filtration en profondeur.

10. Procédé de l'une quelconque des revendications 1 à 9, dans lequel le procédé comprend en outre l'étape consistant à
(i) précipitation de la molécule d'acide nucléique à partir de l'éluant ; *et*/*ou*
(ii) filtration de l'éluant par filtration à écoulement tangentiel pour isoler la molécule d'acide nucléique ; éventuellement dans laquelle la taille moyenne des pores de la membrane de filtration utilisée dans l'étape de filtration à écoulement tangentiel est ≤ 0,2 µm ; *et*/*ou*
(iii) la lyophilisation de la molécule d'acide nucléique purifiée d'intérêt, éventuellement dans laquelle la lyophilisation est effectuée en présence d'un hydrate de carbone ; *et*/*ou*
(iv) recueillir un écoulement après l'étape de mise en contact du mélange avec un matériau échangeur d'anions en présence de la solution comprenant le sel à une concentration qui permet la liaison sélective de la molécule d'acide nucléique au matériau échangeur d'anions ; éventuellement dans lequel l'écoulement comprend de l'ARN, de l'ADN génomique, des protéines, des fractions cellulaires, ou des combinaisons de ceux-ci, de préférence dans lequel l'écoulement comprend de l'ARN ; et/ou
(v) laver le matériau échangeur d'anions avec une solution tampon de lavage avant l'élution de la molécule d'acide nucléique d'intérêt ; éventuellement dans lequel la solution tampon de lavage comprend un sel chaotrope à une concentration inférieure à la concentration requise pour l'élution de la molécule d'acide nucléique d'intérêt, dans lequel le sel chaotrope est de préférence choisi dans le groupe constitué par : le chlorure d'ammonium, le chlorure de potassium, le chlorure de sodium, le sulfate de magnésium, le chlorure de magnésium, le nitrate de magnésium, et leurs mélanges.

11. Procédé de l'une quelconque des revendications 1 à 10, dans lequel le procédé comprend en outre une autre étape de purification suivant l'étape d'élution et éventuellement d'isolement de la molécule d'acide nucléique ;
dans lequel éventuellement la seconde étape de purification comprend l'utilisation d'un milieu de chromatographie d'adsorption aromatique thiophile ayant une sélectivité qui permet de séparer l'ADN plasmidique super enroulé de l'ADN circulaire et/ou linéaire ouvert.

12. Procédé de l'une quelconque des revendications 1 à 11, dans lequel le procédé comprend en outre l'étape consistant à
(i) utiliser l'acide nucléique pour exprimer un polypeptide d'intérêt ; de préférence, l'acide nucléique est un ADN plasmidique ; ou
(ii) l'utilisation de l'acide nucléique pour produire et récolter un polypeptide d'intérêt ; de préférence dans lequel l'acide nucléique est un ADN plasmidique ; éventuellement dans lequel la méthode comprend en outre l'étape de formulation du polypeptide d'intérêt dans une composition pharmaceutique.

13. Procédé de l'une quelconque des revendications 2 à 11, dans lequel le procédé comprend en outre l'étape consistant à utiliser l'ADN plasmidique purifié pour la production d'ARN.

14. Procédé de l'une quelconque des revendications 1 à 13, dans lequel le procédé comprend les étapes de récolte et de lavage des cellules, de lyse des cellules, de neutralisation et d'élimination des floculats avant la mise en contact du mélange résultant comprenant la molécule d'acide nucléique d'intérêt avec le matériau échangeur d'anions.

15. Procédé de l'une quelconque des revendications 1 à 14, dans lequel le procédé ne comprend pas d'étape de précipitation de CaCl₂ pour éliminer l'ARN.
